# EUROPEAN PATENT SPECIFICATION

(11) **EP 1 737 985 B1**
(45) Date of publication and mention of the grant of the patent: **30.12.2015**
(21) Application number: 05817602.5
(22) Date of filing: 07.04.2005
(51) Int. Cl.: C12M 1/34, C12Q 1/68, G01N 33/558

(54) **NUCLEIC ACID DETECTION SYSTEM**
SYSTEM FÜR DEN NACHWEIS VON NUKLEINSÄUREN
SYSTEME DE DETECTION D'ACIDES NUCLEIQUES

(30) Priority: 07.04.2004 US 560197 P; 03.05.2004 US 567845 P
(43) Date of publication of application: 03.01.2007
(73) Proprietor: Access Bio, Inc., Monmouth Jct., NJ 08852 (US)
(72) Inventor: JUNG, Jaean, Monroe Twp., New Jersey 08831 (US); CHOI, Young Ho, Princeton, New Jersey 08540 (US); KIM, Youngsun, East Brunswick, New Jersey 08816 (US)
(74) Representative: Fiener, Josef
(86) International application number: PCT/US2005/011977
(87) International publication number: WO 2006/041524

(56) References cited:
- WO-A1-01/36974
- WO-A1-97/03207
- WO-A1-99/30131
- WO-A1-2004/007078
- WO-A1-2004/099438
- US-A1- 2003 119 042
- US-B1- 6 440 706
- US-B1- 6 649 378
- HAERMAE H ET AL: "Europium nanoparticles and time-resolved fluorescence for ultrasensitive detection of prostate-specific antigen" CLINICAL CHEMISTRY, AMERICAN ASSOCIATION FOR CLINICAL CHEMISTRY, WASHINGTON, DC, vol. 47, no. 3, 1 March 2001 (2001-03-01), pages 561-568, XP002370440 ISSN: 0009-9147

## Description

### BACKGROUND OF THE INVENTION

### 1. Field of the Invention:

The invention relates to the field of high sensitivity detection of nucleic acid molecules by using a lateral flow nucleic acid detection system.

### 2. General Background and State of the Art:

Advances in preventative medicine go a long way towards protecting today's soldiers from diseases such as mosquito-borne dengue fever when the soldiers are deployed to tropical or subtropical areas. Despite precautions taken in the field, however, some soldiers have still contracted dengue fever or the more serious dengue hemorrhagic fever/dengue shock syndrome (DHF/DSS). Rapid field-level detection of the disease is crucial to timely treatment and prevention of further outbreaks.

The family Flaviviridae contains almost 70 viruses including those causing yellow fever, dengue fever, West Nile fever and Japanese encephalitis. Due to increases in global travel, outbreaks of these viruses have begun occurred outside the tropics with greater frequency. In the continental US, outbreaks of St. Louis encephalitis virus and a recent outbreak of West Nile virus which started in New York City and expanded to the whole eastern coast of US. In addition, two mosquito vectors, *Aedes Aegypti* and *Aedes albopictus,* are present in the US and under certain circumstances, each could transmit dengue viruses. According to the CDC, this type of transmission has been detected in south Texas in 1980, 1986 and 1995, and has been associated with dengue epidemics in northern Mexico.

Dengue fever and DHF/DSS have emerged as the most important arthropod-borne viral diseases of humans (Gubler and Clark, Emerg Infect Dis. 1995 Apr-Jun;1(2):5 5-7). There are four distinct dengue virus types (DEN-1, DEN-2, DEN-3, and DEN-4), each capable of causing disease in humans. The conserved 3'-noncoding sequences of four dengue virus serotypes have been successfully utilized to develop a TaqMan-based RT-PCR (funded by MIDRP STO A/L from 2002-2003) to quantitatively identify dengue viruses from different regions of the world.

The polymerase chain reaction (PCR) provides accurate pathogen identification, but requires rigorous sample preparation, complex reactive components of limited shelf life, precise temperature regulation, sophisticated hardware, a complex detection process, and trained personnel, all of which are difficult to secure in the field or in "real-time" conditions such as in the event of a bioterrorist attack.

This is appropriate for laboratory diagnosis, but is of limited utility in "real-time" field conditions where a rapid assessment of the nature and presence of biological contamination is vital to minimizing its impact. A chromatographic lateral assay principle applied as a protein detection method can be developed as a DNA detection system.

The critical link in most detection systems is to elicit a distinctive, detectable signal that is responsive to particular target sequences from the presence of a biological pathogen via non-polymerase chain reaction nucleic acid based approach.

### SUMMARY OF THE INVENTION

The present invention is directed to a signal amplification system such as europium encapsulated microparticles and/or electroconductivity in conjunction with chromatographic lateral flow assay. This system is an improved nucleic acid detection system that retains all of the advantages of conventional immunochromatographic assay: 1) One step 2) Field-usable 3) Utilizes stable reagents 4) No special storage requirements 5) Rapid results. The amplified signal is measured by a small portable reader, which gives quantitative or qualitative results.

The invention is directed to scale up production of a fluorogenic nucleic acid reagent kit for serotype-specific target nucleic acid detection. The invention is further directed to lyophilized kit components, which are sensitive, specific and stable. Various concentrations of cultured pathogens such as viruses which include dengue virus or dengue virus cDNA may be detected.

In one embodiment, the inventive system employs ready to use lyophilized reagent pads for gene amplification. Reaction matrix may include buffers, dNTP's, RNAase inhibitor, reverse transcriptase, labeled specific primers such as dengue virus serotype specific primers (DEN-1, -2, -3, -4), lower primers, Biotin dUTP, Taq polymerase, internal control mRNA, and control mRNA specific primers. Biotin dUTP is added to label the nucleic acid amplified product in order to detect using a lateral flow assay and to amplify the indicator signal. Fluorogenic primers are designed to increase their fluorescence intensity when incorporated into the double-stranded PCR product (Nazarenko et al., Nucleic Acids Res. 2002 May 1;30(9):e37) and to apply the PCR products to the lateral flow immunochromatographic assay to identify the dengue serotype.

In a preferred system of the invention, a time-resolved fluorescence technology is used, preferably a system based on europium embedded micro particles conjugated with anti-hapten antibody and time-resolved confocal scanning device for signal detection. This feature provides additional sensitivity of assay. This highly sensitive chromatographic lateral flow signal amplification system requires less threshold cycle number (C^{t}) than the fluorescence detection system. The inventive PCR product detection system is rapid (<30 min), a one step format, and stable (storage at <30 degrees C for more than 1 year). The assay is easy to operate, inexpensive, portable, uses heat-stable reagents, and has no special storage requirements. These features make it a fast and easy, ready-to-use RT-PCR kit in real time conditions by untrained personnel.

The inventive detection system may also be applied to detect any organism, including but not limited to pathogens such as militarily significant virus, including Flavivirus, such as Japanese Encephalitis virus, Yellow Fever virus, West Nile virus, small pox and so on. Other pathogens include bacteria, such as *Bacillus anthracis.*

The present invention is also directed to non gene amplification based target nucleic acid based detection by using Europium based and/or electroconductivity based methods of target nucleic acid detection.

The inventive nucleic acid based detection system amplifies a signal from the low concentration of specific genomic DNA sequences from biological pathogens without rigorous sample preparation, complex reactive components of limited shelf life, sophisticated hardware, a complex detection process, or trained personnel. This method is also unique, specific, simple, and the amplifying system is easy to operate in a field-deployable detection module.

In a particular exemplification of the invention, the invention is directed to a real-time Reverse Transcriptase Polymerase Chain Reaction (RT-PCR) technology of the dengue 3'-noncoding region based assay system into ready-to-use dengue virus detection and diagnosis system. A field deployable and user-friendly diagnostics device using lateral flow immunochromatographic assay is shown with and without real-time fluorogenic thermal cycler. The present invention allows dual application to the real time PCR and/or conventional PCR.

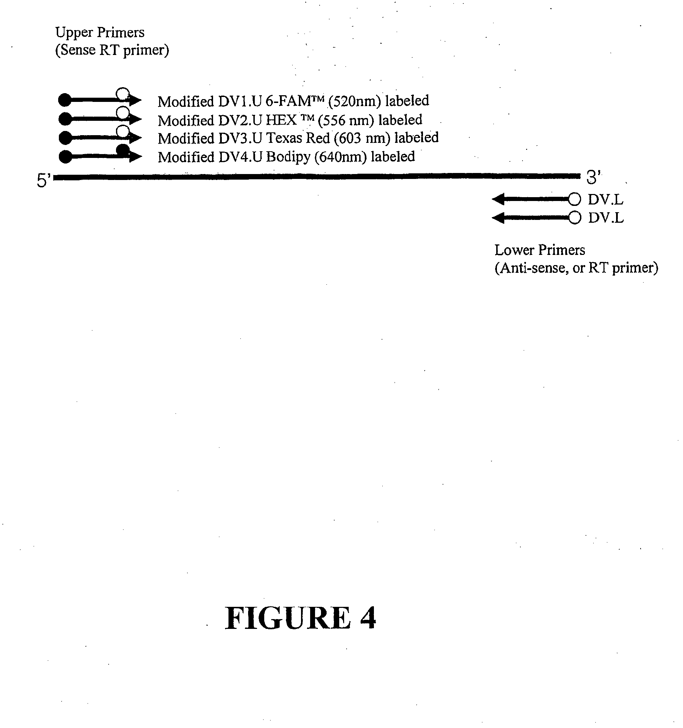

The invention is further directed to the detailed analysis result of serotype specific information in conjunction with the RT reactions. Multiplex PCR reaction may be carried out in a single tube (Fig. 4). All reaction components for RT reaction and PCR reaction are lyophilized in the matrix with proper formulation. Biotin dUTP or labeled oligonucleotide primers and fluorescent labeled beacon primers are added to label the PCR product and to detect using real time fluorogenic thermal cycler or lateral flow detection kit. Four serotypes of dengue virus can be identified in a single PCR reaction. This reaction kit can be stored at room temperature for more than one year.

It is to be understood that any suitable detection system may be used, including fluorescent, chemiluminescent, enzymatic or any other dye based system, so long as the binding of the probe to the target nucleic acid in a sample can be detectably assayed using the inventive lateral flow system. Such a dye system may include a molecular beacon type system with fluorescein based label, or it may contain other labels such as a biotin label with a colloidal gold conjugated avidin or streptavidin partner or any other detectable conjugable chemical such as a lanthanide element such as europium. Other types of detection labels and methods are within the purview of the invention.

The invention is further directed to a self-performing device. This rapid nucleic acid detection system contains all reagents and components in precise quantities to generate test results after sample addition. The system also may also have a built in heating pad to precisely match nucleotide target sequence identification based on probe oligonucleotide composition.

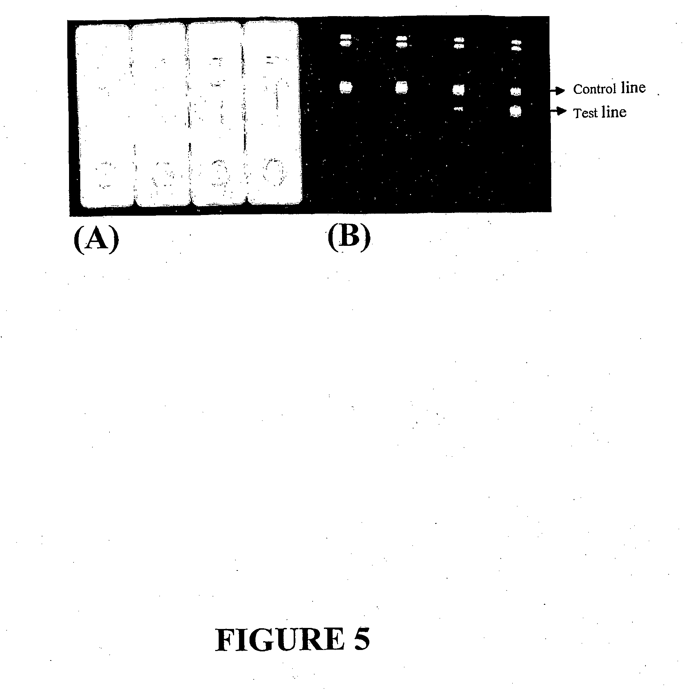

The target DNA of biological pathogen in the sample may react with the europium particle-labeled oligonucleotides, the europium particle-labeled Peptide Nucleic Acids (PNAs) with Light Addressable Direct Electrical Readout (LADER), or electro conductivity. This europium labeled oligomer and electroconductivity detection method working in conjunction in a lateral flow assay system can increase sensitivity 10 to 1,000 times compared with colloidal gold microparticle. (Fig. 5) These two signal amplification systems can increase sensitivity higher than 103target/100µl (= 10 aM). Additionally, combining these detection systems with chromatographic lateral flow assays can increase the sensitivity by more than 10 times compared to an equilibrium plate assay (Hampl etal. Anal Biochem 2001; 176-187).

In one aspect, the invention is directed to a lateral flow device for detecting target nucleic acid comprising: a reservoir area, a dye area and a test area, wherein the reservoir area comprises ready to use nucleic acid amplification mix comprising primer labeled with a hapten and/or dNTP labeled with a hapten; the dye area comprises a specific binding partner to a first type of hapten linked to a reporter dye; and the test area comprises a specific binding partner to a second type of hapten. The device may contain a reservoir with more than one different second type of hapten where multiple forms of the target nucleic acid is being detected. The first type of hapten may be biotin, and its specific binding partner may be streptavidin, and the reporter dye may be europium or gold. Further, the second type of hapten may be biotin, fluorophore, or oligopeptide, and its specific binding partner may be streptavidin or an antibody specific to the flurophore or oligopeptide if a single form of the target nucleic acid is being detected. Further, the second type of hapten may be multiple types of fluorophores or oligopeptides and its specific binding partner may be an antibody specific to each type of fluorophore or oligopeptide. The source of the target nucleic acid may be a pathogen, which may be a virus such as dengue virus and the serotypes of the dengue virus particle may be detected by using different second types of hapten. Also, the primer may be molecular beacon type.

The present invention is also directed to a method of assaying for the presence of a target nucleic acid in a sample, comprising contacting the sample to the reservoir area of the device according described above, wherein if the target nucleic acid is present in the sample, the target nucleic acid is amplified and labeled with at least two types of hapten, and is transported to the dye area where the first hapten is bound to its specific binding partner linked to a reporter dye, and is further transported through the test area by capillary action, and is bound to the second type of hapten-specific binding partner on the test area, the detection of which indicates that the target nucleic acid is present in the sample. The reservoir may include more than one different second type of hapten where multiple forms of the target nucleic acid is being detected. The first type of hapten may be biotin, and its specific binding partner may be streptavidin, and the reporter dye may be europium or gold. The second type of hapten may be biotin, fluorophore, or oligopeptide, and its specific binding partner may be streptavidin or an antibody specific to the flurophore or oligopeptide if a single form of the target nucleic acid is being detected. The second type of hapten may be multiple types of fluorophores or multiple types of oligopeptides and their specific binding partner may be an antibody specific to each type of fluorophore or oligopeptide. The source of the target nucleic acid may be a pathogen.

The present invention is further directed to a method of assaying for the presence of a target nucleic acid in a sample, comprising contacting the sample to a reservoir area of a lateral flow device wherein if the target nucleic acid is present in the sample, the target nucleic acid is amplified and labeled with at least one type of hapten, and is transported to a dye area where the hapten is bound to its specific binding partner linked to a reporter dye, and is further transported through the test area by capillary action, and is bound to target specific oligonucleotide in the test area, the detection of which indicates that the target nucleic acid is present in the sample, wherein the detection is by light addressable direct electrical readout or by detection of the reporter dye. The hybridization in the test area may be controlled by built in heating pad in the lateral flow device. The hapten may be biotin, and its specific binding partner is streptavidin, and the reporter dye may be europium or gold.

The invention is also directed to a method of assaying for the presence of a target nucleic acid in a sample, comprising contacting a sample to a reservoir area of a lateral flow device wherein if the target nucleic acid is present in the sample, the target nucleic is transported through the test area by capillary action and is bound to target specific oligonucleotide labeled with Europium. Further, the target nucleic acid may be amplified before being transported to the test area. Amplification may not be necessary if using as sensitive an assay where electroconductivity and europium detection are being used.

### BRIEF DESCRIPTION OF THE DRAWINGS

The present invention will become more fully understood from the detailed description given herein below, and the accompanying drawings which are given by way of illustration only, and thus are not limitative of the present invention, and wherein;
Figures 1A and 1B show A. modified serotype-specific upper primers; and B. hairpin structure made using the primers.
Figure 2 shows structure of molecular beacon dengue virus serotype specific upper primers.
Figure 3 shows lower primers for PCR anti-sense primer and RT reaction primer.
Figure 4 shows diagram of multiplex PCR amplification.
Figures 5A-5B show the inventive assay system. 5(A) shows the test device of the system before testing. The device has at least two main parts, Sample well at the bottom part of device and result reading window at the middle of the device. 5(B) shows the test results after assay. Two lines indicate a positive and one line a negative. The control line serves as an internal built-in control. It should appear if assay is performed properly.
Figure 6 shows a multiplex PCR product identification assay.
Figure 7 shows a picture of PCR product identification device.
Figure 8 shows a configuration of the lateral flow device.
Figure 9 shows a structure of 5' amino modifier linked type 2 forward primer
Figure 10 shows a configuration of immunochromatographic PCR product identification assay with labeled PCR product (strip format).
Figure 11 shows a configuration of immunochromatographic PCR product identification assay with labeled PCR product (device format).
Figure 12 shows a structure of 5' biotinylated forward primer.
Figure 13 shows a structure of 5' rhodamine red labeled reverse primer.
Figure 14 shows a structure of 5' synthetic oligopeptide labeled forward primer.
Figure 15 shows an assay procedure for the strip test.
Figure 16 shows an assay procedure for device format assay.
Figure 17 shows comparison results of real-time PCR and rapid PCR product analysis kit for Dengue virus serotype 1 detection. Both tests performed with 1.5mM MgCl₂. **Lane A:** 4,340,000 copies/test, **Lane B:** 868,000 copies/test, **Lane C:** 173,600 copies/test, **Lane D:** 34,720 copies/test, **Lane E:** 6,944 copies/test, **Lane F:** 1,388 copies/test, **Lane G:** 277 copies/test, **Lane H**:55copies/test, **Lane I:** No template
Figure 18 shows comparison results of real-time PCR and rapid PCR product analysis kit for Dengue virus serotype 2 detection. **Lane A:** 3,720,000 copies/test, **Lane B:** 1,240,000 copies/test, **Lane C:** 413,000 copies/test, **Lane D:** 138,000 copies/test, **Lane E:** 45,900 copies/test, **Lane F:** 15,300 copies/test, **Lane G:** 5,100 copies/test, **Lane H:** 1,700 copies/test, **Lane I:** 567 copies/test, **Lane J:** 189 copies/test, **Lane K:** 63 copies/test, **Lane L:** 21 copies/test, **Lane M:** No template
Figure 19 shows comparison results of real-time PCR and rapid PCR product analysis kit for Dengue virus serotype 3 detection. Both tests performed with 1.5mM MgCl₂, **Lane A**: 5,090,000 copies/test, **Lane B:** 1,018,000 copies/test, **Lane C:** 203,600 copies/test, **Lane D:** 40,720 copies/test, **Lane E:** 8,144 copies/test, **Lane F:** 1,628 copies/test, **Lane G:** 325 copies/test, **Lane H:** 65 copies/test, **Lane I:** No template
Figure 20 shows principles of Light Addressable Direct Electrical Readout (LADER).
Figure 21 illustrates a schematic of the reader device and a blow-up of the contact head for the CBT chips in EDDA format.
Figure 22 shows the electrochemical behavior of an electrode modified with 20 nucleotide long capture probe and covalently attached Os-label before and after hybridization with the matching target that itself is modified with a ferrocenium (FcAc) label.
Figure 23 shows a configuration of self-performing rapid nucleic acid detection system using electroconductive assay.
Figure 24 shows a diagram of assay principle for detection of biological pathogen specific DNA sequence using time-resolved fluorescent emission and/or conductivity change detection.
Figures 25A-25B show configurations for heating system.
Figures 26 A and B show a configuration for a heating system.
Figures 27 A and B show a configuration for a heating system.
Figure 28 shows a diagram of a test device with places for instrument connector.
Figure 29 shows gene point of care testing (GPOCT) device, which is an integrated DNA sampler.
Figure 30 shows a detailed embodiment of the GPOCT device.
Figure 31 shows a detailed embodiment of the GPOCT device.

### DETAILED DESCRIPTION OF THE PREFERRED EMBODIMENTS

In the present application, "a" and "an" are used to refer to both single and a plurality of objects.

As used herein, "multiple forms of target nucleic acid" refers to multiple isoforms or serotypes of a target nucleic acid, such as dengue virus, which may have four serotypes.

As used herein, "nucleic acid" or "oligonucleotide" as the terms are used in the probe setting refers to any string of nucleotides. Thus, DNA, RNA or PNA are included.

As used herein, "nucleic acid amplification" refers to polymerase chain reaction (PCR), ligase chain reaction (LCR), Qβ replicase, strand displacement assay (SDA), nucleic acid sequence-based amplification (NASBA), or cascade rolling circle amplification (CRCA).

As used herein, "ready to use" in the context of nucleic acid amplification refers to all of the necessary reagents and enzymes to carry out the target nucleic acid amplification upon contact with the sample that includes the target nucleic acid.

In one aspect, the invention is directed to raising signal for nucleic acid detection. The method may comprise using a variety of labels or haptens to sensitively measure the signal.

In one aspect of the invention, the ready-to-use nucleic acid amplification pre-mix may be included in a container. The sample may be added to the pre-mix and the nucleic acid amplification carried out. The pre-mix may contain reagents for nucleic acid amplification including primers that are differentially labeled at the 5' and/or 3' ends. For real-time PCR application, the 5' and 3' ends of a single primer may be differentially labeled in the molecular beacon style with a fluorophore at one end and a quencher at the other end. Each of these labels may be considered haptens as antibodies against these labels are available and may be generated. Further, the body of the amplified product may be optionally labeled with a hapten labeled dNTP, such as biotin. The pre-mix may be dried or freeze-dried, and the pre-mix may be suitable for amplification by isothermal amplification PCR and/or real time PCR.

For lateral flow assay, the haptenized or labeled amplified nucleic acid or non-amplified genomic DNA containing the target nucleic acid (usually cut) that is able to flow to the dye area is contacted on the lateral flow assay device at the reservoir area, which contains reagents that are suitable for hybridization conditions or antibody/antigen binding conditions or other specific binding pair conditions.

The nucleic acid may then travel to the dye area, which contains a specific binding partner to the target nucleic acid complex of nucleic acid and the haptens that are attached to the nucleic acid. Thus, the specific binding partner may be a target-specific oligonucleotide, an antibody to one of the haptens or a high affinity ligand such as streptavidin, which binds with high affinity to biotin. The specific binding partner may be linked or conjugated to a dye, preferably a highly sensitive dye, such as europium or gold to form a complex of target nucleic-specific binding partner-dye. Alternatively, the specific binding partner may be further haptenized with a different label. Further, for the oligonucleotide specific binding partner, the label may be europium, fluorophore, FITC, biotin, oligopeptide and so on, so long as the label is detectable by any means.

As the complex moves to the test area, the test area may be immobilized with another specific binding partner to one of the other haptens in the target nucleic acid complex such as an oligonucleotide specific for the target nucleic acid, whereupon the binding of the complex to the different specific binding partner on the test area indicates the presence of the target nucleic acid. The oligonucleotide that is used in the dye area and immobilized in the test area may be a peptide nucleic acid or RNA. In the case of a DNA oligonucleotide immobilized on the test area, hybridization of the target nucleic acid complex with the oligonucleotide on the test area generates an electroconductivity signal, which can be measured by the highly sensitive LADER assay. Further when the europium labeled oligonucleotide is hybridized to the oligonucleotide or PNA immobilized on the test area, highly sensitive assay is made which combines the sensitivities of LADER assay and europium assay. For instance, if europium labeled portion at one is bound then europium will yield a strong signal. If the europium labeled area is not bound to the immobilized nucleic acid, but only the non-europium labeled part of the nucleic acid is bound, then the LADER conductivity is available as an alternative assay.

In another aspect of the invention, the test area may be immobilized with a target specific oligonucleotide and/or at least one other specific binding partner for one of the haptens to further differentiate and specify the target nucleic acid. Thus, in one specific embodiment, a specific binding partner to one of the haptens on the target nucleic acid such as specific to either the 5' label or the 3' label may be immobilized in the test area, and an oligonucleotide probe to the target nucleic acid may also be immobilized so that detection the target nucleic acid is made with greater specificity.

The description below exemplifies detecting dengue virus using the nucleic acid amplification method of PCR, however, it is understood that any pathogen may be detected using any chemically driven nucleic acid amplification method such as PCR, LCR, NASBA and so on.

### Reverse Transcriptase Polymerase Chain Reaction (RT-PCR)

The reverse transcriptase polymerase chain reaction (RT-PCR) provides accurate viral pathogen identification, but as used conventionally currently requires rigorous sample preparation, complex reactive components of limited shelf life, precise temperature regulation, sophisticated hardware, a complex detection process, and trained personnel. This is appropriate for laboratory diagnosis, but is of limited utility in "real-time" field conditions.

The inventive system optimizes and scales up production of the RT-PCR reagent kits for generic and serotype-specific microorganism detection, including dengue virus detection. Special freeze-drying protocol is developed to generate dried and ready-to-use dengue assay kits that contain all the components for RT reaction multiplex PCR reaction in a single tube. The kits can be used in either general thermal cyclers or real time PCR thermal cyclers such as fluorogenic PCR thermal cyclers.

### Extraction of viral RNA

Viral RNAs are extracted from virus suspensions of the virus infected sera, such as dengue-infected sera according to QiAamp Viral RNA Handbook (Qiagen Inc. Valencia, CA 91355).

### Design synthesis of molecular beacon serotype specific primers

Molecular beacons are "hairpin" oligonucleotides that form a stem-and-loop structure and possess an internal reporter signal, such as fluorescent reporter and quencher molecule. When they form a hairpin structure, the fluorescent reporters and the quencher molecules are close together and the fluorescence is suppressed. When they bind to complementary target sequence, they undergo a conformational transition that switches on their fluorescence (Bonnet et al., Proc Natl Acad Sci USA. 1999 May 25;96(11):6171-6.). The hairpin molecular beacon can be used as a fluorogenic PCR primer or a fluorogenic probe. When the oligonucleotide primer is modified to form hairpin structure with fluorophore, this primer can provide a low initial fluorescence of the primers that increases up to 8-fold upon formation of the PCR product (Nazarenko et al., Nucleic Acids Res. 2002 May 1;30(9):e37). The hairpin oligonucleotides may be as efficient as linear primers and provide additional specificity to the PCR by preventing primer-dimers and mispriming.

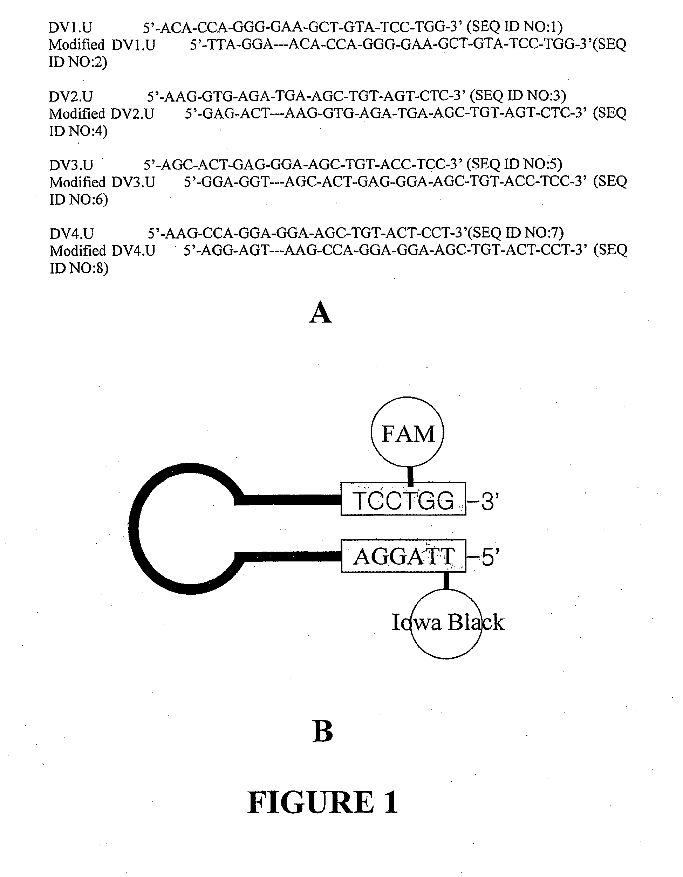

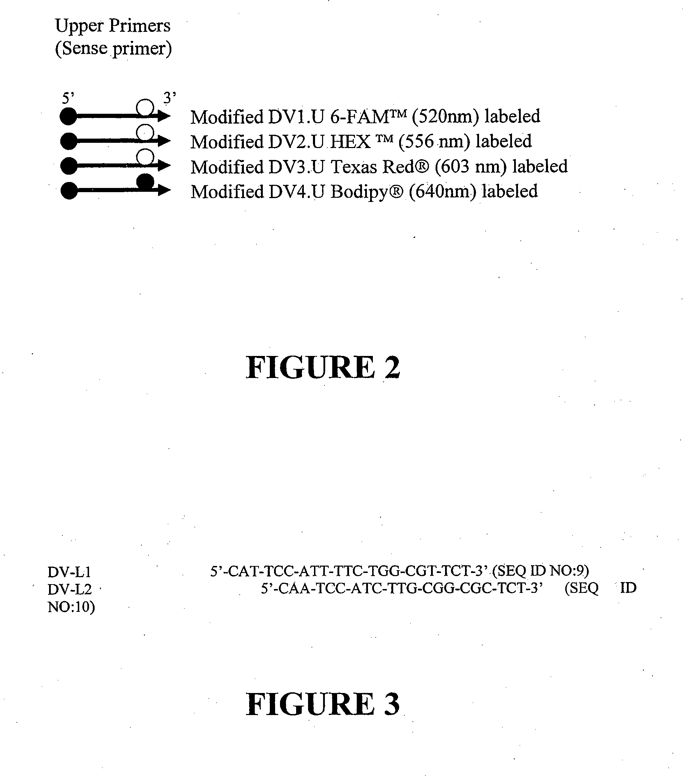

Based on this previous study, upper primers may be modified to form a hairpin structure (Fig. 1). To make a hairpin primer, 5' tail is extended that is complementary to the 3' end of the primer. The 5' tail forms a blunt-end hairpin at temperature below its melting point. Reporter fluoresceins (6-FAM™ (520 nm), HEX™ (556 nm), Texas Red® (603 nm), Bodipsy® (640 nm), etc) are labeled to the third or fourth base (T) from the 3' end. Quencher fluorescein (Iowa black™ quenching range from 520nm to 700nm)) is labeled to the extended 5' end, complementary sequence to the 3' end. All reporter dyes and quenchers are available from Integrated DNA Technology, Inc. (Coralville, IA) and Molecular Probes, Inc. (Eugene, OR). Expected secondary structure and fluorescent labeled positions are shown in Fig. 2.

In another aspect of the invention, the fluorescein also functions as a hapten antigen to react with anti-hapten antibody to identify serotype specific PCR products using lateral flow rapid one step identification test kit.

### Design synthesis of anti-sense primers and RT primers

A generic anti-sense primer DV-L1 (nucleotide residue 368-388) with good homology among 3'-noncoding sequences of dengue 1-3 are designated as the RT primer for these viruses. Even though the dengue 4 genome shares significant homology with DV-L1 sequence, there are five nucleotide mismatches within the DV-L1 primer as shown in Fig. 3. Therefore, a separate anti-sense primer DV-L2 is specifically used in the RT reaction for dengue 4 virus detection. These two anti-sense primers (DV-L1 and DV-L2) are used as the generic RT primer set to transcribe RNA for all four dengue virus serotypes (Houng et al., 2001 J. Virol. Meth. 95:24-35).

**Multiplex RT-PCR reaction in a single tube** The RT reactions and multiplex PCR reaction may be carried out in a single tube (Fig. 4). All reaction components for RT reaction and PCR reaction are lyophilized in the matrix with proper formulation. Biotin dUTP and labeled oligonucleotide primers and fluorescent labeled beacon primers are added to label the PCR product and to detect using real time fluorogenic thermal cycler or lateral flow detection kit. Four serotypes of dengue virus can be identified in a single PCR reaction. This reaction kit can be stored at room temperature for more than one year.

### PCR result identification

A commercially available real time fluorogenic PCR system can be used for this ready-to-use kit. Depending upon the fluorescence detection system, a ready-to-use kit can be adjusted for customization. In addition, this kit can be used under non-ideal laboratory conditions without a real time thermal cycler.

The lateral flow immunochromatographic assay is an alternative system to identify amplified PCR products. This method is unique, specific, and easy to operate in a field-deployable system. This system works for virtually all biological pathogens and viruses and provides a secure result in the field or in "real-time" conditions such as in the event of a U.S. soldier deployment to a dengue endemic area.

### Non PCR lateral flow nucleic acid detection

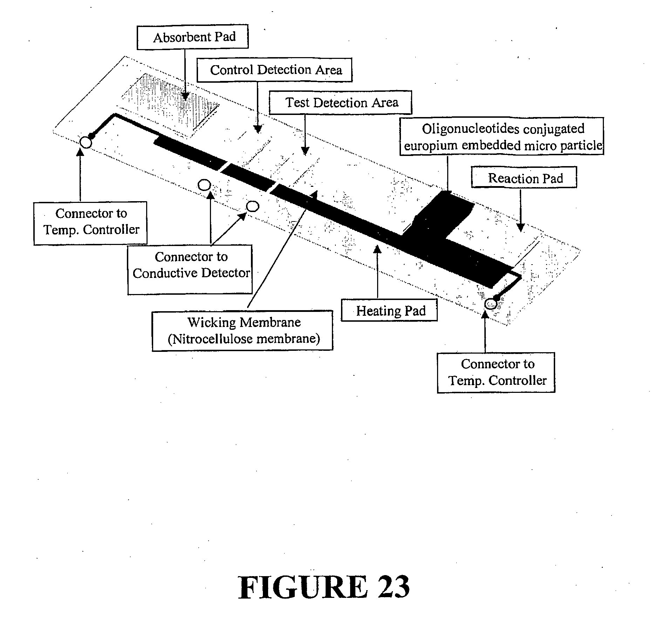

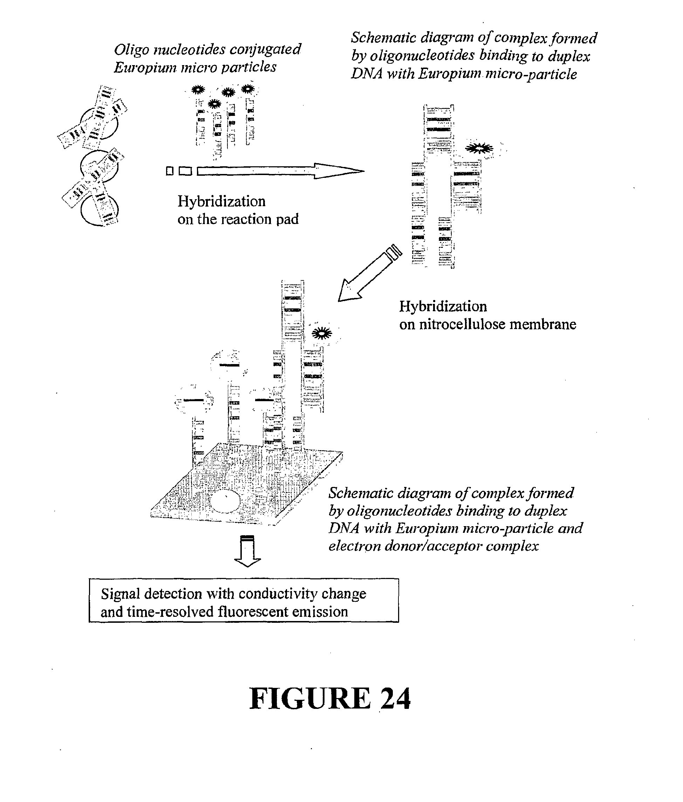

A configuration of a rapid, self-performing nucleic acid detection system is shown in Fig. 23, which contains all reagents and components in precise quantities to generate test results after sample addition. The system also has a built in heating pad to precisely match nucleotide target sequence identification based on probe oligonucleotide composition. The assay principle is described in the diagram (Fig. 24). The sample passes first through the reservoir pad that contains buffer to optimize the pH of the sample, detergents to suspend all components in the sample, and a porous filter to generate proper flow through the device. The sample subsequently flows, by capillary action, to the dye area where target DNA of biological pathogen in the sample react with the europium particle-labeled oligonucleotides or Peptide Nucleic Acids (PNAs). The reaction complex thus formed then migrates through the wicking membrane where capture oligonucleotides embedded in the conductive test and control area. Initial europium labeled probe reaction allows opening the target nucleotide sequence.

### Lateral Flow Assay with Time-Resolved Fluorescence

Ultra-sensitive time-resolved fluorescent (TRF) dye is used in the lateral flow assay format. This ensures improved sensitivity while maintaining the beneficial aspects of a lateral flow assay. Nanosized TRF dye contains 30,000-2,000,000 europium molecules entrapped by β-diketones, which possess one of the highest quantum yields of the known lanthanide chelators (Harma, Technology Review 126/2002, TEKES, National Technology Agency, Helsinki 2002). This encapsulation has substantially no effect on the fluorescence efficiency of the dye. For a typical 100 nm size europium particle, the fluorescence yield is equivalent to about 3,000 molecules of fluorescein. By comparison, phycobiliprotein B-PE (perhaps the most fluorescent substance known) has a fluorescence yield equivalent to about 30 fluorescein molecules. Since a 100 nm particle is about 10 times the diameter of phycobiliprotein B-PE and a thousand times greater in volume/mass, Seradyn europium particles are 100 times more fluorescent than B-PE on a molar basis, but only 10% as fluorescent on a volume/mass basis (Seradyn, Color-Rich™ Dyed and Fluoro-Max™ Florescent Microparticles Technical Bulletins 1999). The lateral flow assay with this TRF dye particle can increase detection sensitivity up to pg/ml level which means that threshold cycle number (Ct) can be decreased up to 10 cycles. This feature provides additional specificity.

The system requires a simple and self-performing one-step method that does not require complicated and expensive automated instrument. An example of a small plastic piece that contains an inventive self-performing assay strip and its test outcome is shown in Figure 5.

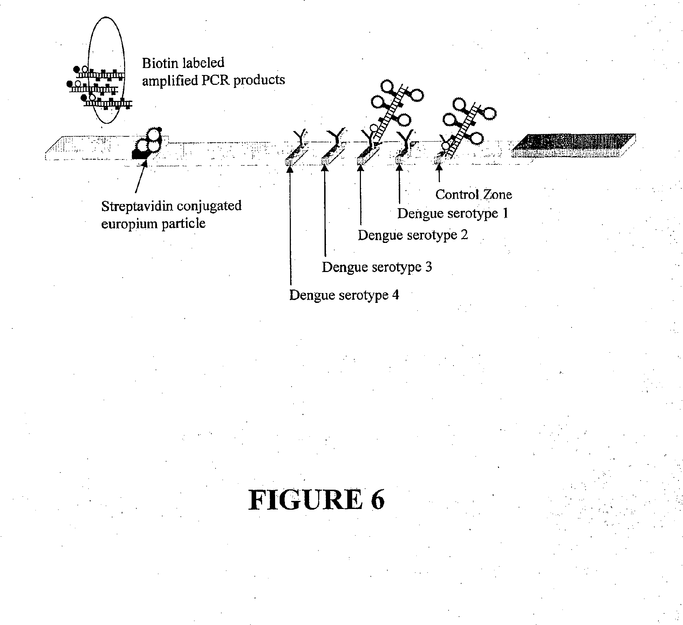

Multiple analyte detection with single sampling and no further procedural steps is possible with the assay system provided herein. An immunochromatography assay system is preferred. If various antibody-dye conjugates are embedded in the dye pad area and antibody specific for each reporter dye is respectively immobilized in separate zone on membrane, each test line will provide distinctive information for each specific dengue serotypes (Fig. 6).

### Field deployable detection system

Field deployable detection system is contemplated. A strip reader may be used to detect the presence of the sample. For example, an instrument for measuring time-resolved lanthanide emission may be used, which is equipped with a nitrogen laser, a diffraction grating spectrometer, a charge coupled device (CCD) and a mechanical chopper for time gating.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. The following examples are offered by way of illustration of the present invention, and not by way of limitation.

### EXAMPLES

Below is a stepwise procedure exemplifying a dengue virus cDNA for positive control and rabbit globin mRNA for internal control. The overall design of amplified PCR product identification system is described.

### Example 1

### Design of molecular beacon primers

Molecular beacon primers using Beacon Designer 2 (Bio-Rad) primer and probe design software are designed.

### Preparation of dual labeled molecular beacon primers

Various reporter fluoresceins (Oregon Green® (517nm), 6-FAM™ (520 nm), HEX™ (556 nm), TAMRA™ (573nm), Texas Red® (603 nm), Bodipsy® (640 nm), etc) are labeled to the third or fourth base (T) from the 3' end. Quencher fluorescein (Iowa black™ quenching range from 520nm to 700nm)) are labeled to the extended 5' end, complementary sequence to the 3' end. All reporter dyes and quenchers are available from Integrated DNA Technology, Inc. (Coralville, IA) and Molecular Probes, Inc. (Eugene, OR).

### Internal quality control system

The ready-to-use kit contains 1ng of rabbit globin mRNA and each of two globin-specific PCR primers as an internal reaction control. The PCR product of rabbit globin mRNA is detected by lateral flow identification kit at the control zone. This control zone intensity can be used as a reference for a semi quantitative result.

### Ready-to-use RT-PCR reaction kit

Various matrix media such cellulose, glass fiber, polyester or rayon and so forth are evaluated. The formulated components listed below are dried under a constant vacuum in a lyophilizer. The matrix is selected with respect to optimal reagent reconstitution and RT-PCR reaction.

The list of formulated components:
RT-PCR buffer,
Proper concentration of MgCl₂
dNTP's
Biotin dUTP (Biotin-16-dUTP or Biotin-21-dUTP)
RNAase inhibitor
reverse transcriptase
labeled dengue virus serotype specific primers (type 1, 2, 3, 4)
labled lower primers
rabbit globin mRNA
two globin-specific PCR primers
Taq polymerase
Stabilizers (RNase/DNase free BSA and sugar, etc.)

The main purpose of this step is to find the optimal freeze-drying and key components storage conditions combined with release of all components and reconstitution under RT-PCR reaction. Various combinations of the chemicals such as but not limited to buffers, detergents, sugars and polymers and biological materials such as proteins and other biological polymers are tested to find the best conditions. Ready-to-use RT-PCR kit is tested by a iCycler IQ™ (BioRad) and ABI 7700 real-time fluorogenic thermal cycler. MgCl₂ concentration is a significant factor for a successful lateral flow assay. The preferred range for MgCl₂ concentration is not higher than about 1.5 mM as above a certain amount of MgCl₂ primer dimmers and false positive results are seen. Addition of Biotin dUTP increases sensitivity because of either more capturing or more indicator binding capability (Table 1). Substrate inhibition was overcome by controlling the labeled and unlabeld ratio and applying excessive capture and indicator amount.

Table 1 shows comparison data between amplified PCR results with labeled primers and amplified PCR result with labeled primers and biotin dUTP incorporation for Dengue virus serotype 2 detection.

**Table 1. Comparison data between Dengue virus amplified PCR results**

| Copy Number/Test | Labeled primers | Labeled primers and Biotin dUTP incorporation |
|---|---|---|
| 1000/test | ++ | +++ |
| 200/test | + | ++ |
| 50/test | +/- | + |

### Example 2

### Lateral flow rapid PCR product identification test kit

### Configuration of the test

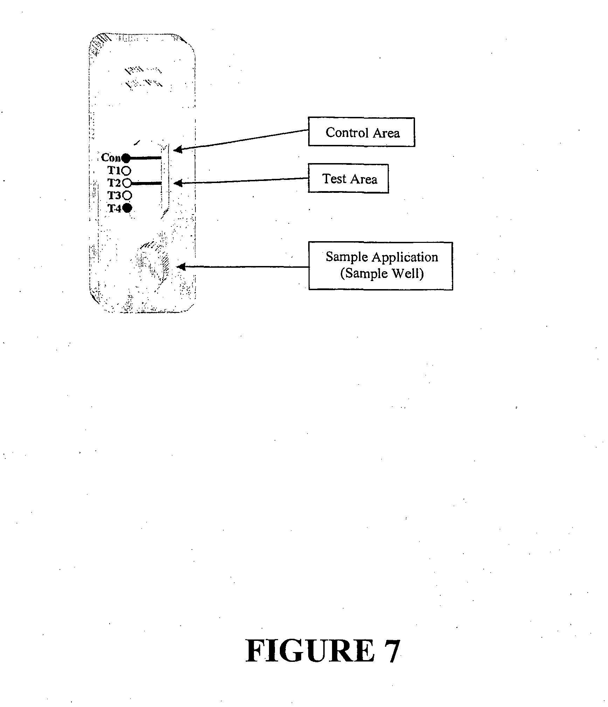

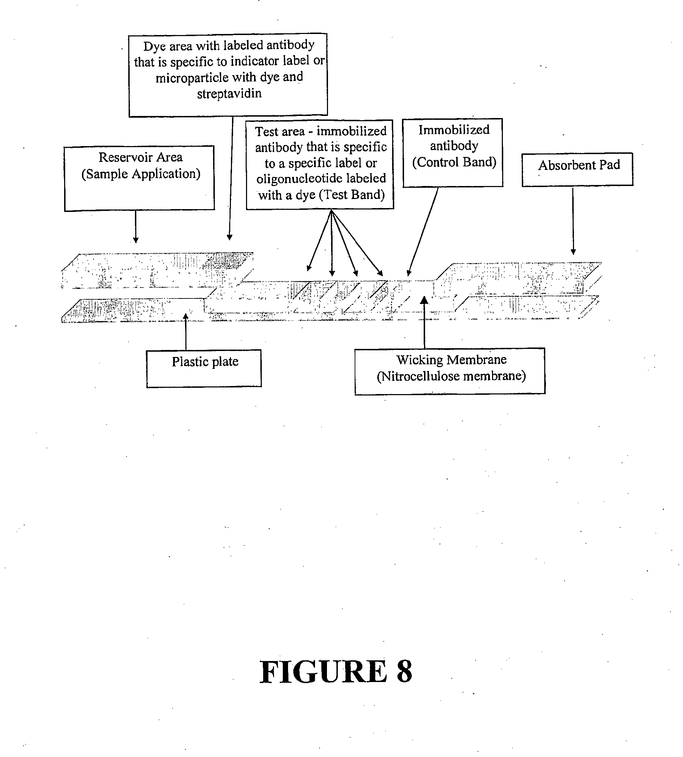

The inventive test kit is designed to be a self-performing device. Lateral flow rapid PCR product identification test kit such as depicted in Fig. 7 contains all reagents and components in precise quantities at the reservoir pad to generate test results after sample addition. The assay principle and configuration is described in Figure 8. The sample passes first through a reservoir pad that contains buffer to optimize the pH of the sample, detergents to suspend all components in the sample, separation column materials such as sepharose beads, and so on to separate un-reacted substrates such as labeled primers and biotinylated dUTP and a porous reservoir to generate proper flow through the device. The sample subsequently flows, by capillary action, to the dye area where serotype specific PCR products of dengue virus DNA in the sample react with the europium particle-labeled streptavidin. The reaction complex thus formed then migrates through the wicking membrane embedded with anti-fluorescent dye antibody test area as well as a control area.

The test may be performed on-site by a person with minimal training and rapid results are available within 10 minutes after adding one or two drops of DNA extracted sample to the disposable test card. The labeled streptavidin and target DNA complex is captured by test and control areas. Test area distinguishes each serotype specific PCR products and this area can be detected by a reader equipped with a time-resolved fluorescence scanning device when Europium is used as the signal detection label. A separate control area detects any amplified PCR product as an internal quality control system. This control area reaction assures that the key test components are functioning properly.

### Preparation of capture anti-dye antibody immobilization

Antibodies to fluorescent dyes provide unique opportunities both for signal enhancement and for secondary detection of PCR products labeled with fluorescent dyes. Various anti-fluorophore antibodies are available from Molecular Probes, Inc. (Eugene, OR). Each antibody is printed using a printing machine (BioDot Corp.) at various concentrations between 0.5 and 2 mg/ml with a thickness of 0.5 to 1 mm. The quality of the visible bands and binding characteristics of antibodies can be checked by Ponceau's method (Hassan, J., et al, J. Clin. Lab. Immunol., 24:104, 1987).

### Preparation of anti-hapten antibody conjugated microparticle

### Europium particles

Various particle-sizes (50nm - 300 nm) of europium particles were purchased from Seradyn (Indianapolis, Indiana) and Molecular Probes (Eugene, Oregon). The carboxyl group of europium chelate nanoparticles were activated with 10 mmol/L N-(3-dimethylaminopropyl)-N'-ethylcarbodiimide and 100 mmol/L N-hydroxysulfosuccinimide for 30 min. The activated particle was washed once with 50 mM MES buffer, pH 6.1. Antibody or streptavidin was added 5 mg/L. After 2 hour incubation, the antibody or streptavidin coated particles were washed three times with 50 mM MES buffer, pH 6.1.

### Gold particles

Various particle-size (20nm-200nm) gold particles were purchased from British Biocell International (UK). To optimum conjugation, pH of the colloidal gold solution was adjusted to 6.5. Antibody or streptavidin was added 5 mg/L. After 15 minute incubation, 2 g/L of bovine serum albumin was added. The antibody or streptavidin coated particles were washed two times with 10 mM phosphate buffer, pH 7.4.

### Optimization of microparticle conjugation to streptavidin

The effects of the concentration of streptavidin, blocking formulation of the conjugate and storage of conjugate are measured under various conditions. The covalent cross-linking is also determined. Conjugation yield and scale-up feasibility are important decision factors in selecting an optimized system.

### Optimization of microparticle conjugation to anti-hapten antibody

The effects of the concentration of the anti-hapten antibodies, blocking formulation of the conjugate and storage of conjugate are measured under various conditions. The covalent cross-linking is also determined. Conjugation yield and scale-up feasibility are important decision factors in selecting an optimized system.

### Kit Formulation

### Dye area

The main purpose of this step is to find optimal dye-streptavidin or hapten-antibody conjugate storage conditions combined with release of the labeled streptavidin or the antibody under wet assay conditions. Various combinations of chemicals such as but not limited to buffers, detergents, sugars and polymers and biological materials such as proteins and other biological polymers are tested to find the best conditions.

### Reservoir area

Since the sample is applied to the reservoir area, the sample is conditioned to optimal condition for assay with various combinations of the chemicals and biological materials.

### Wicking Membrane

Since this component facilitates the capillary migration of the nucleic acid sample, it should efficiently mobilize nucleic acid, allow immuno-reaction between the labeled target nucleic acid bound to the capture streptavidin and provide strong capillary action for a prolonged time period. Finding optimum concentrations of detergent with proper stabilizers is also important.

### Lateral flow assay

Configuration of this system is similar to the antigen-antibody imnumo-chromatographic assay, but streptavidin is conjugated to the colloidal gold particle and the signal can be generated by streptavidin-biotin interaction. Serotype 2 specific primer was labeled with fluorescent hapten such as rhodamine red. Anti-rhodamine red antibody was immobilized onto the nitrocellulose membrane. The sample passes first through the reservoir pad and flows, by capillary action, to the dye area where serotype specific PCR products of dengue virus react with the colloidal gold conjugated streptavidin. The reaction complex thus formed then migrates through the wicking membrane where anti-fluorescent dye antibody embedded test and control area.

Lateral flow dengue virus serotype 2 tests were successfully applied to detect and identify dengue virus serotype 2 specific PCR amplification. The detection limit of dengue virus serotype 2 test was < 100pg/test, corresponding to < 1 femto mole/test. Theoretically, this sensitivity is enough to detect amplified PCR products from single digit copies of dengue virus cDNA.

The reported dengue virus serotype 2 tests can be performed on-site by a person with minimal training and rapid results are available within 10 minutes after adding PCR products to the disposable test card. Also, this system is a simple and self-performing one-step method that does not require complicated and expensive laboratory conditions.

### Example 3

### Preparation of fluorecein isothiocyanate (FITC) conjugated oligonucleotide

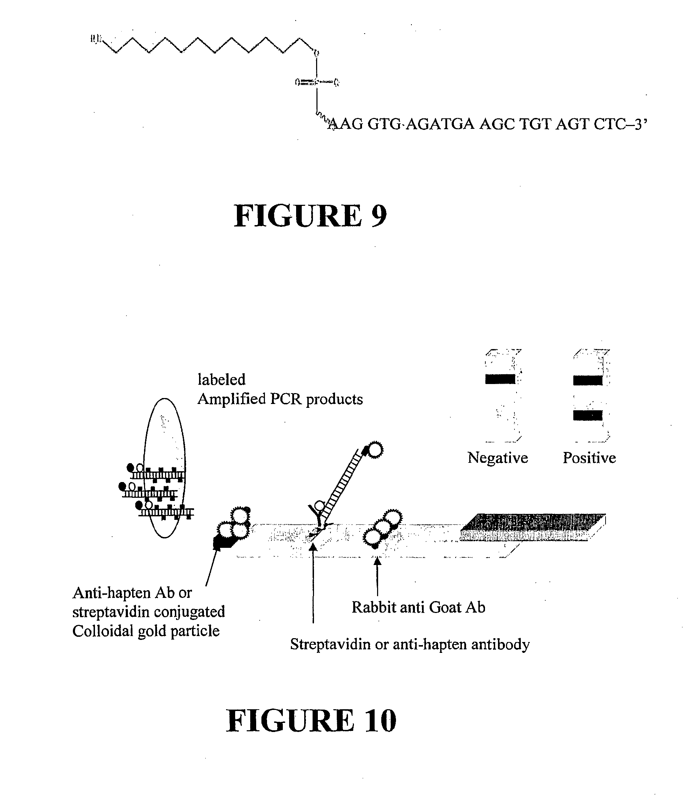

Fluorecein isothiocyanate (FITC) was purchased from Sigma. For the conjugation, 5' of dengue virus type 2 forward primer was modified with twelve-carbon chain (C12) amino modifier linker. (Fig. 9) FITC was attached to the modified oligomer using conventional procedure.

### Preparation of lateral flow assay

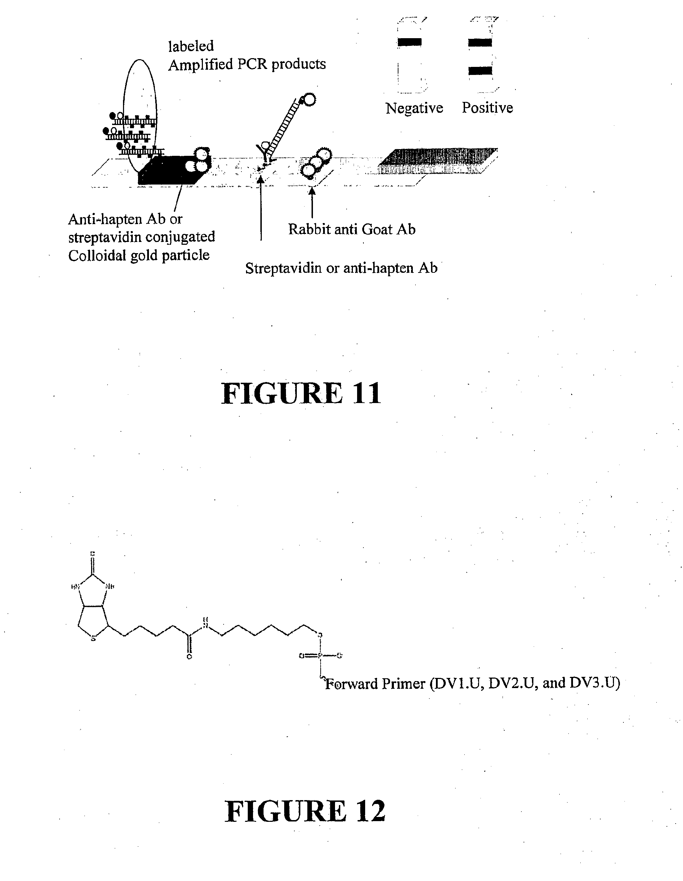

Streptavidin is conjugated to the colloidal gold particle and the signal can be generated by antigen-antibody reaction. Biotin molecule is conjugated at the 5'end of reverse primer (DV. L1). FITC molecule is conjugated at the 5' end of forward primer (DV2.U). Amplified PCR products with those modified forward and reverse primer contain both biotin and FITC molecules. Biotin dUTP is incorporated during the PCR amplification. When this sample is applied to the testing strip, biotin tags of amplified PCR products react with colloidal gold conjugated with streptavidin. And then the PCR product-gold conjugate complex migrate through the nitrocellulose membrane by chromatographic capillary power. The complex is captured by the immobilized anti-FITC antibody. Depending on the amount of captured gold or europium particle, visible signal is generated (Fig.10). Device format construction is shown in Fig. 11.

### Preparation of biotin labeled forward primers of Type 1, Type 2, and Type 3

Biotin molecule was conjugated at the 5' end of Dengue virus forward primers (DV1.U, DV2.U, and DV3.U). Each forward primer was modified with six carbon chain (C6) linker at the 5' (Fig. 12).

### Preparation of rhodamine red labeled reverse primer (DV. L1)

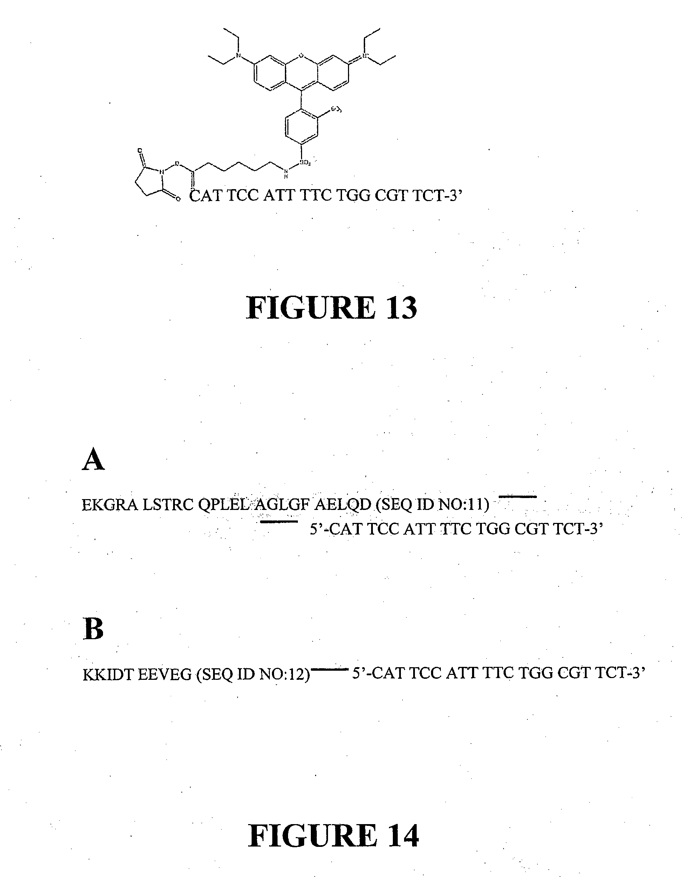

Reverse primer was modified with rhodamine red molecule conjugated at the 5' end of reverse primer (DV.L1) (Fig. 13).

### Preparation of oligopeptide labeled reverse primer (DV2. U)

Forward primer was modified with synthetic oligopeptide molecule conjugated at the 5' end of the primer (DV2.U) (Fig. 14).

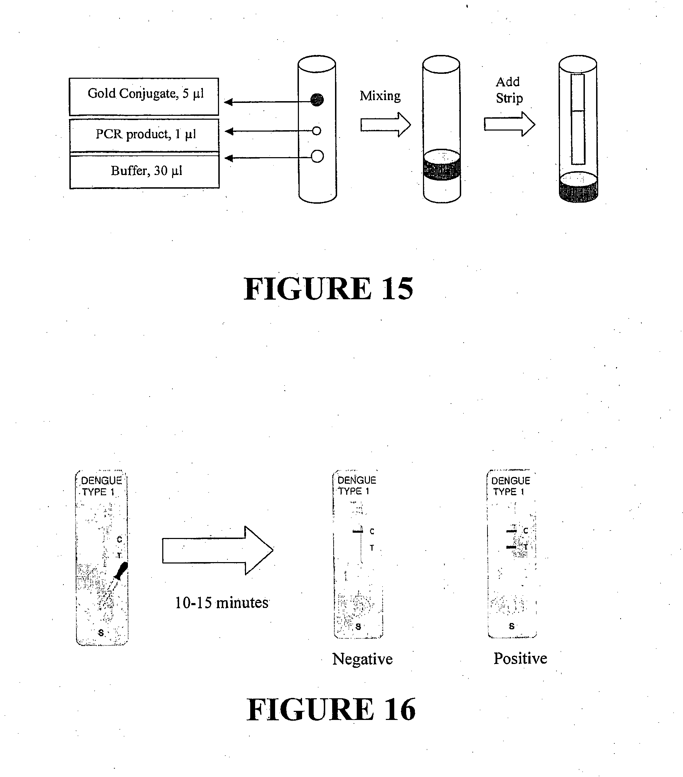

### Assay procedure for the strip format assay (Fig. 15)

### 1) Sample application

Amplified PCR product at the various template concentrations, gold or europium conjugate and buffer are mixed, and are contacted added on a nitrocellulose membrane.

### 2) Assay buffer

PBS containing detergent was prepared following conventional methods.

### 3) Assay

Read test result after 10 minute

### Assay procedure for the device format assay (Fig. 16)

### 1) Sample application

Amplified PCR products at the various template concentrations were added on the sample well, such as 2 µl of PCR product.

### 2) Assay buffer

PBS containing detergent was prepared using conventional methods and a couple of drop of a developer solution specific for the signal detection label are contacted with the device on the nitrocellulose.

### 3) Assay

### Read test result after 10 minute

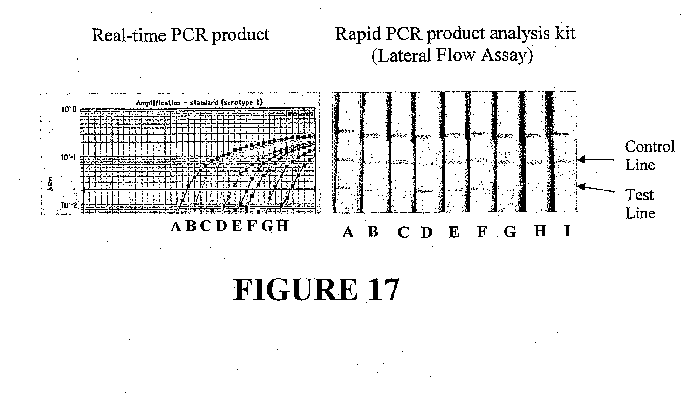

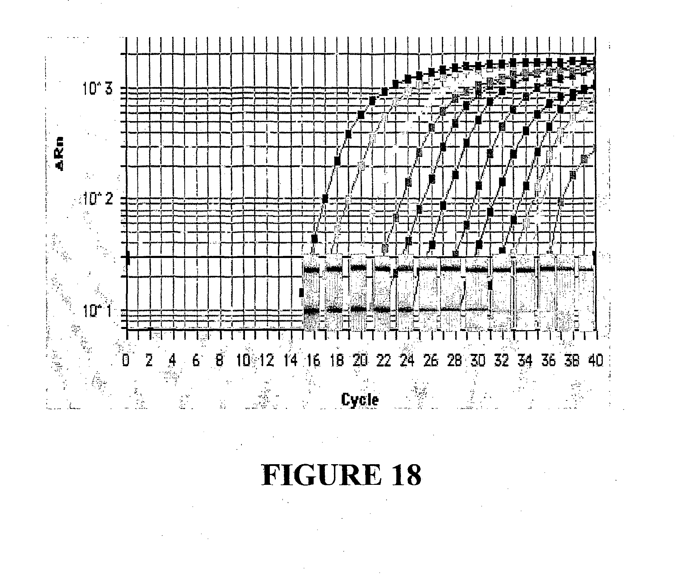

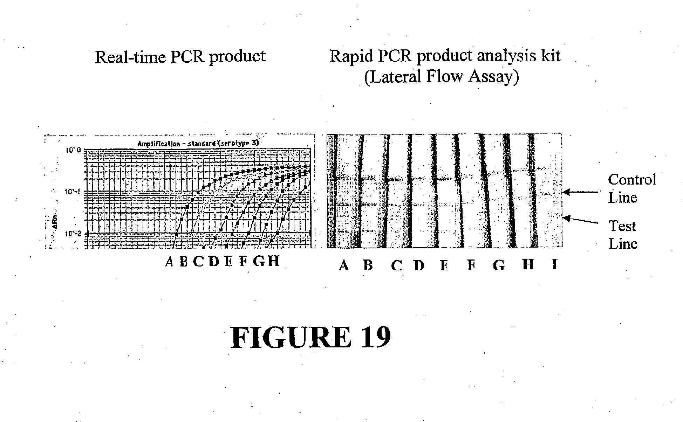

Fig. 17, 18, and 19 present comparison results of real-time PCR and rapid PCR product analysis kit for Dengue serotype 1, 2, and 3.

### Example 4

### Lateral flow assay with electroconductivity and europium labeled oligonucleotides

Light Addressable Direct Electrical Readout (LADER)'s principle is based on the difference in conductivity of oligonucleotides in single strand ("isolating") and double strand ("conducing") form. This conductivity difference can be regarded as a switch. A molecular electrical circuit is build up by connecting the capture probes to an electrode, attaching an electron-donor/-acceptor complex (DA) and adding a dissolved active component. The electron-donor/-acceptor complex acts as a second, light induced switch. Incident light induces a charge transfer from D to A and from there the electron can be transferred to the electrode, if the probe is hybridised. Charge refilling of the DA-complex by the active component closes the circle and continuous cycling generates a highly amplified read-out signal. LADER principle is described in EP 1144685 B1, which describes protecting photosynthetic reaction centers and similar systems as electrochemical complexes for the LADER technology, and DE 19921940 C2.

Donor/acceptor complex works well in pigment/protein complex known in photosynthesis as a reaction centre, capable of transferring an electron from the porphyrin-donor to the chinon-acceptor within 100ps after light induction. This pigment/protein complex can be easily divided into the chinon-acceptor and the remaining (apo-)protein. The chinon-acceptor is modified in a way that it can be covalently attached to the oligonucleotide capture probe on the one hand and to the (apo-)protein on the other hand after reconstitution of the apo-protein to the probe-bound chinon-acceptor.

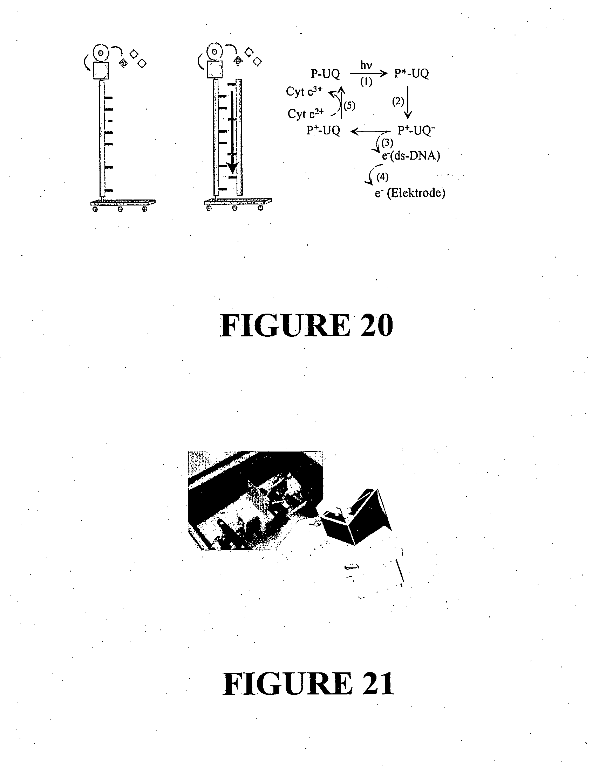

The approach is described in the reaction scheme of Figure 20. The ubiquinone (UQ) depleted photosynthetic reaction centre is reconstituted and crosslinked to UQ that is bound to double stranded DNA. The donor is represented by the porphyrin system P while the ubiquinone moiety forms the acceptor A. Illumination in a first step (1) creates an excited state P*-UQ which proceeds in a further step (2) to the temporarily stable charge separated state P⁺-UQ⁻. From this state an electron is withdrawn over the DNA (3) to the electrode (4) oxidising P⁺-UQ⁻ to P⁺-UQ and resulting in the measurable anodic photocurrent. The cycle back to the initial state is closed by the reducing agent cytochrome c²⁺ (X) which itself is oxidised by reducing (5) the system to P-UQ.

The readings may be read and interpreted and are identified qualitatively and quantitatively by automatic comparison between the reference and measurement signals at each individual spot. Figure 21 illustrates a schematic of the reader device and a blow-up of the contact head for the CBT chips in EDDA format. The reader is partially developed and capable of all liquid handling steps necessary to perform by a simple "press bottom" approach. Reference measurement data (before hybridization) of the bar-coded chips are stored in an internal buffer and compared with the actual measurement data after hybridization. The difference value per electrode is displayed on an external computer screen and can be further processed.

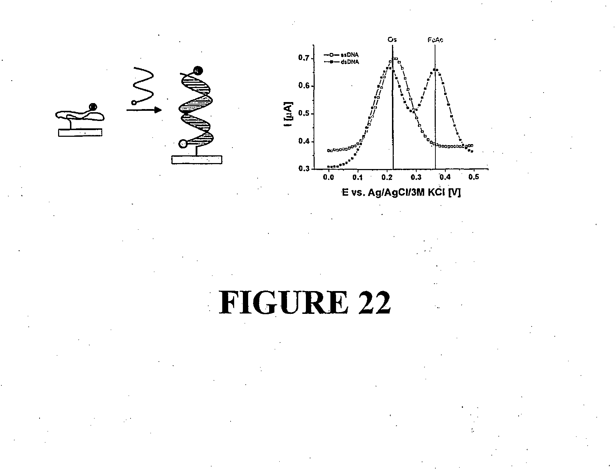

The conductivity of double stranded DNA vs. single stranded DNA and the associated tunnelling parameter β was further probed by determination of the electron transfer rate constant for DNA single and double strands of varying length with electrochemical impedance spectroscopy. Figure 22 shows the electrochemical behavior of an electrode modified with 20 nucleotide long capture probe and covalently attached Os-label before and after hybridization with the matching target that itself is modified with a ferrocenium (FcAc) label. In the single strand situation there is an electrochemical response attributable to the Os-label at the remote end of the capture probe as expected. After hybridization an additional peak shows up that is due to the ferrocenium label (FcAc-Peak). This demonstrates not only that the Os-label at the remote end of the capture probe can be electrochemically scanned, but also that the efficiency of hybridization is close to 100% (the two peaks of the labels representing capture probe and target are almost identical in height).

### Chromatographic assay with conductivity detector

Configuration of self-performing rapid nucleic acid detection system (Fig. 23) contains all reagents and components in precise quantities to generate test results after sample addition. The system also has a built-in heating pad to precisely match nucleotide target sequence identification based on probe oligonucleotide composition. The assay principle is described in the diagram in Fig. 24. The sample passes first through the reservoir pad that contains buffer to optimize the pH of the sample, detergents to suspend all components in the sample, and a porous filter to generate proper flow through the device. The sample subsequently flows, by capillary action, to the dye area where target DNA of biological pathogen in the sample react comprising europium particle-labeled oligonucleotides. The reaction complex thus formed then migrates through the wicking membrane where capture oligonucleotides are embedded suitable for conductive test and control area. Initial europium labeled probe reaction allows for the opening of the target nucleotide sequence.

The proposed test may be performed on-site by a person with minimal training and rapid results are available within 30 minutes after adding one or two drops of DNA extracted sample to the disposable test card. The labeled oligonucleotide probe and target DNA complex is captured by conductivity labeled test and control area. Test area displays target sequence and this area can be detected by a reader equipped with a time-resolved fluorescence scanning device and/or conductivity reader. A separate control area displays counter oligonucleotides sequences of europium-labeled oligonucleotides. This control area reaction assures that the key test components are functioning properly.

The chromatographic lateral flow assay is an amplification system. The target DNAs in liquid sample are steadily concentrated by electric affinity to the charged testing area on a membrane when they are moving through the membrane by capillary power. Even where molecules exist at very low concentration, the actual concentration of the molecule in the test area is much higher than the one in the sample.

### Built in heating pad

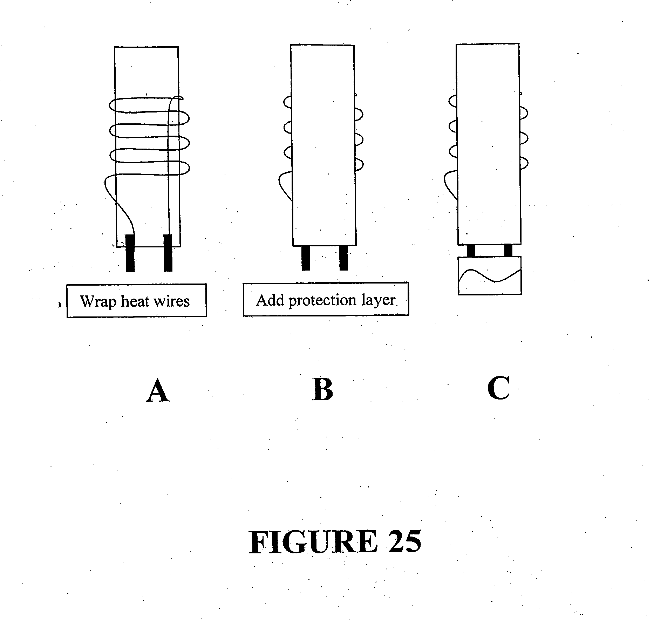

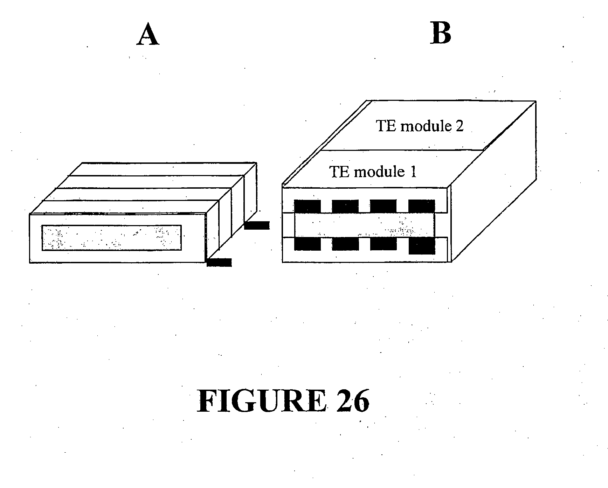

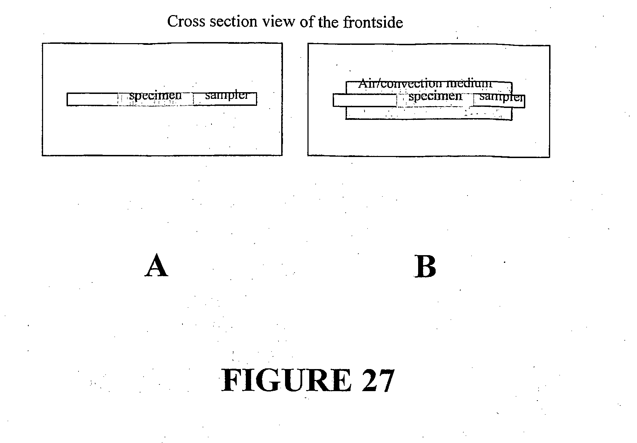

Lateral flow assay can be mounted on the heating PCB/PCP (printed circuit paper), which is printed high resistance metal (e.g. Nickel/Chrome alloy is available for heating purpose) on a base board. This system could have relatively precise temperature monitor and control at low price. Sample configurations are shown in Figs. 25A-25C, Figs. 26A-26B, and Figs. 27A-27B).

### Diagram of Testing Device

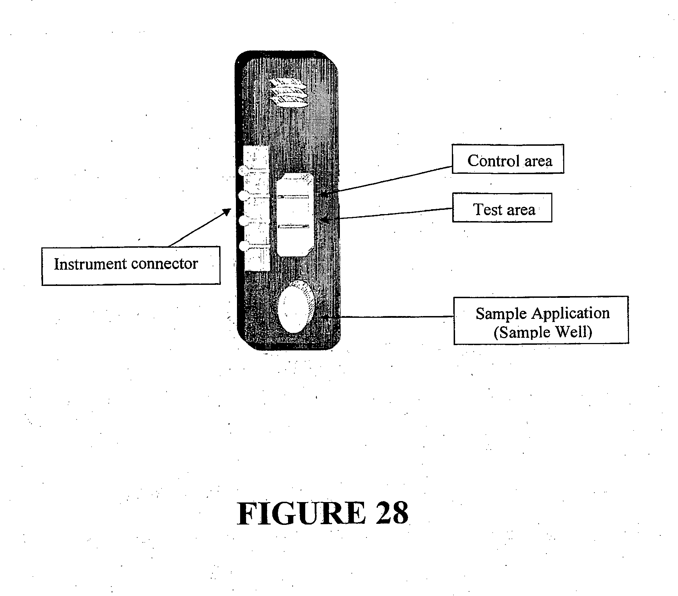

A test kit is made of a disposable plastic case with two windows, one to view the control and test area, and the other providing a well to receive the sample as shown in Fig. 28.

Inside the device is a test strip with two pads. The first pad contains buffers, detergents, and chemicals to ensure consistent test results, and also it contains target oligonucleotides conjugated europium particles in a specially formulated buffer system. The second pad is for removing the excess fluid that has already passed through the reaction membrane. Two types of oligonucleotides are separately immobilized on the test and control area of the nitrocellulose membrane as thin lines. The oligonucleotides specific to the target biological pathogens are immobilized in the test area, while the other oligonucleotides specific to europium labeled probe are immobilized in the control area. Instrument connectors are also included so that instruments for measuring time-resolved fluorescence or conductivity in situations where conductivity labels are used can be linked to the device.

### Example 5

### Lateral flow assay with electroconductivity with europium labeled peptide nucleic acids (PNAs)

All of the methods and procedures described in Example 4 using nucleic acid as the probe for detecting target DNA is applicable for using peptide nucleic acid as the probe as well, including the use of various heat pads and test devices described therein.

### Example 6

### Detection of HLA-DQα sequence polymorphism

The HLA-DQα2 genetic locus can be used for forensic analysis of individual identification as a genetic marker. A variety of analytic techniques have been used to detect genetic variation in PCR-amplified DNA.

Various haptens or fluorescent haptens may be labeled at the primers and probes. Accordingly, various sequences and size of oligopeptides may be used as a hapten; other small molecules, which may act as an epitope, may be used as a hapten, and various fluorescent haptens such as those listed in Table 2 may be used.

**Table 2**

| | Emission Maxima for Reporter Dyes |
|---|---|
| Oregon Green^{®} 488-X | (517 nm) |
| G-FAM Fluorscein | (520 nm) |
| Rhodamine Green™ -X | (527 nm) |
| Oregon Green^{®} 514 | (530 nm) |
| TET™ | (536 nm) |
| JOE | (547 nm) |
| HEX™ | (556 nm) |
| Cy3™ | (563 nm) |
| TAMRA™ | (573 nm) |
| Rhodamine Red™-X | (580 nm) |
| ROX™ | (602 nm) |
| Texas Red^{®}-X | (603 nm) |
| Bodipy^{®}630/650-X | (640 nm) |
| Bodipy^{®} 650/665-X+ | (660 nm) |
| Cy5™ | (662 nm) |
| Cy5.5™ | (707 nm) |

Anti-hapten antibodies are used as a counter part of the above haptens. Many anti-hapten antibodies are commercially available from such companies as Molecular Probes, Inc. Furthermore, anti-hapten antibodies may be immobilized on to a solid matrix or conjugated with various particles. By "various particles", it is meant colloidal gold, latex particles, magnetic or paramagnetic particles, europium embedded latex particles and so on.

### Example 7

### Gene Point of Care Test

In another embodiment of the invention, the invention is directed to a gene point of care testing device (GPOCT), which purpose is to provide a one step or simple step integrated genetic analysis system. The absorbent pad is connected to a matrix, which is connected to a reaction pad, wherein the captured antibody or antigen is immobilized on the matrix as a line or dot lateral flow assay for gene point of care test device. On the reaction pad is a reagent zone where the reaction sample (amplified sample) is applied and migrates to the next media.

### Embodiment 1 - Sample extraction system.

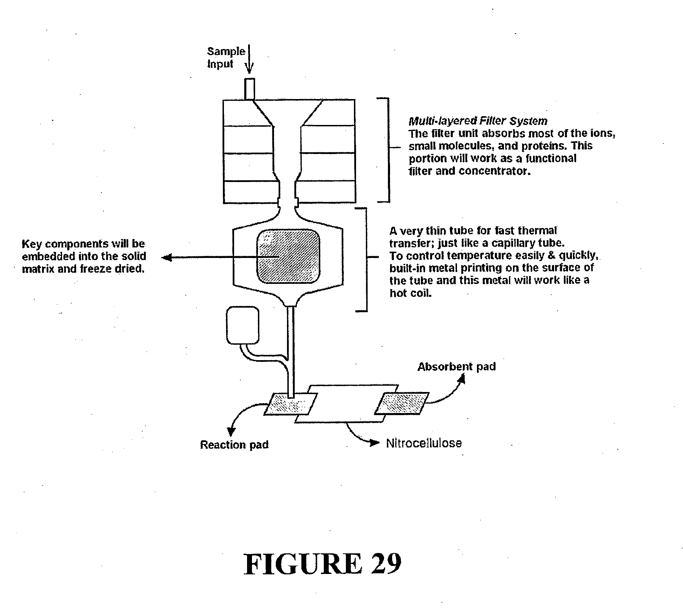

Referring to Fig. 29, a non-limiting example of an extraction system may include:

Reservoir system to remove solution and buffer components to concentrate DNA or RNA samples, and may comprise multi layer absorbent with special treatment, and extraction solution containing without limitation detergent and alkaline buffer.

This buffer can destroy the cell wall or membrane and expose genetic samples like DNA or RNA into the solution. It is contemplated that a protease may be added to remove histone like protein and cell walls. Alternatively, a separate solution capsule or multilayer film may be used.

Further various kinds of reservoir, carbon filter, ion exchange filter may be used. Moreover, the filter may be a semipermeable membrane with a molecular cutoff ranger from 60,000 to 300,000 D or higher and may be non-stick coated.

The filter system removes all potential inhibitors, small molecules, ions, and proteins. As a result, sample can be concentrated 10 to 1000 times from original extraction solution.

### Embodiment 2 - Integrated system from crude sample or extracted DNA sample.

As shown in Figs. 29, the integrated system comprises a sample input area, which is connected to multi-layered filter system, in which the filter unit absorbs most of the ions and small molecules and proteins. This portion works as a functional filtration and concentrator. The filter system is further connected to a very thin tube for fast thermal transfer just like a capillary tube. To control temperature easily and quickly, built-in metal printing on surface of the tube may work like hot coil. Inside the thin tube may be an area for housing certain components embedded into the solid matrix and freeze-dried. The thin tube is connected to a reaction pad, which is further connected to nitrocellulose membrane, and which is further connected to an absorbent pad.

### Embodiment 3-Amplification and Detection Part Overview.

Purified sample is applied, and in an alternative embodiment, the sample is applied and passed through a roller system, which controls the rate of flow of the sample. The sample then passes through a thermal sensor and heat control circuit, and passes through an amplification chamber, which may be comprised of 1. pad containing all required reagents; 2. freeze-dried preformulated reagent pad; and 3. for PCR reaction, this chamber (pad) contains Taq polymerase, dNTP, primers (tagged), other labeling monomer and so forth.

As the sample passes out of the amplification chamber, it is contacted with a reaction pad of the lateral flow assay. While on the lateral flow assay, the sample flows through a result window, wherein the result can be displayed by dot or line or any kind of detectable signal and flows on to absorbent pad.

Parts of the apparatus of the invention are described in detail herein below.

### 1. Thermal sensor and heat control circuit unit.

Various kinds of conductive ink can be printed out on the surface of flexible matrix such as polyester, polycarbonate or polystyrene. Alternatively, various kinds of conductive ink such as carbon, carbon/silver blend, silver, silver/silver chloride, gold, platinum, UV cured dielectric, heat cured dielectric conductive ink may be printed on the substrate and generate proper heat to perform thermocycling variable temperature range from 30°C to 100°C. Thus, temperature sensor and temperature control can be achieved by detecting and controlling their current and resistance.

Thermocycling may be achieved by setting two or three different temperatures and cycling at the temperature repeatedly 20-40 times. Thermocycling condition needs to be optimized depending on their primer sequence.

### 2. Amplification pad

The amplification pad contains all reagents for isothermal amplification such as PCR or RFPCR. All reagents may be premixed and optimally formulated and freeze-dried. By all reagents, it means essential components for the amplification reaction. Such reagents may contain without limitation, following components: reverse transcriptase, DNA polymerase, Taq polymerase, PNAs primers, labled primers, dNTPs, labeled dUTP, buffers, and stabilizers such as proteins, BSA (Bovine Serum Albumin), and EDTA. The amplification pad further may be reconstituted with specific volume of sample application. Primers may be immobilized with glass bead or latex bead to enhance amplification reaction and prevent substrate inhibition. By "substrate inhibition", it is meant that non-reacted primer can inhibit the reaction between anti-tag antibody and polymerized primer. By "primer", it is meant oligonucleotide or PNA (peptide nucleic acid) and oligonucleotide hybrid. By "glass bead" or "latex particle" it is meant microparticles. Microparticles may be available in a size range of 0.01-10.0 µm in diameter. Preferably, the diameter for this embodiment of the invention may be in a range of 0.1-10.0 µm.

U. S. Patent No. 6,153,425 discloses a detection system, however, the '425 patent fails to disclose or suggest that a microparticle in the amplification pad may be used for preventing substrate inhibition.

### Gene Chip

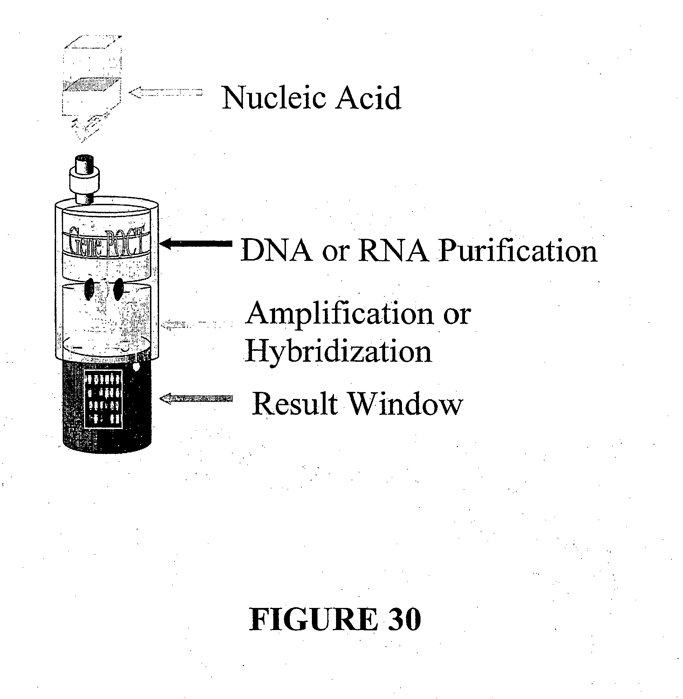

In another aspect of the gene point of care testing device, the invention is directed to a protein chip and gene chip. Regarding the gene chip, particular embodiments have been discussed in describing the gene point of care testing device. In a preferred embodiment, the device may comprise a nucleic acid extractor connected to a DNA or RNA purification chamber, which is further connected to an amplification or hybridization chamber, which is connected to a result window to view the signal generated (Fig. 30). The gene point of care testing device may incorporate the testing strip described above.

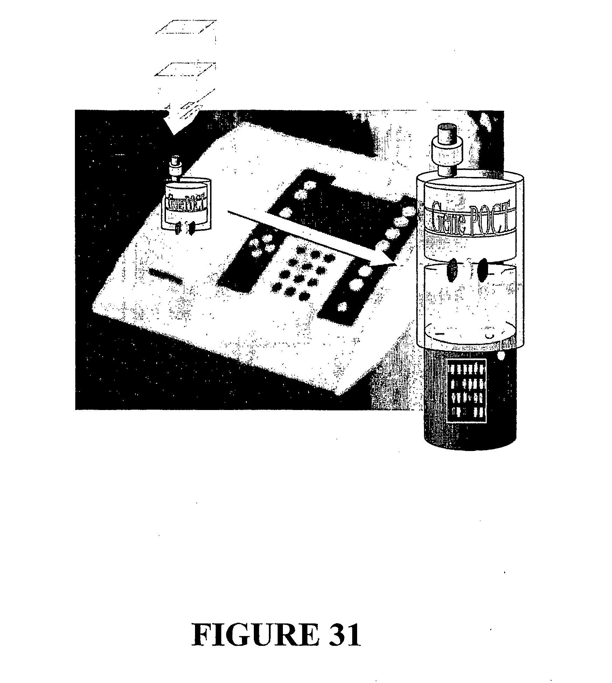

In an embodiment of the invention, a sample is added into an extraction buffer container. Extracted DNA sample may be further added to Gene POCT, and the results may be read in 30 minutes by naked eye or a signal reader (Fig. 31). Gene POCT method is rapid and highly sensitive.

## Claims

1. An immunochromatographic system for detecting one or more target nucleic acids said system comprising:
a container comprising a nucleic acid amplification mix comprising
a primer labeled with a hapten or different haptens at its 5' and 3' ends, and
dNTP labeled with a signal amplifying hapten to form one or more nucleic acid complexes; and
a lateral flow test device comprising
a reservoir area comprising reagents that are suitable for binding of a specific binding partner with the one or more nucleic acid complexes;
a dye area, adjacent to the reservoir area, comprising a specific binding partner to a hapten on the primer or the hapten on the one or more nucleic acid complexes, wherein the specific binding partner is linked or conjugated to a reporter dye or another hapten; and
a test area, adjacent to the dye area, comprising a different specific binding partner specific to a different aspect of the one or more nucleic acid complexes

2. The system according to claim 1, wherein the nucleic acid amplification mix is dried or freeze-dried and is suitable for isothermal amplification, PCR and/or real time PCR.

3. The system according to claim 1, wherein the nucleic acid amplification mix comprises multiple types of hapten where multiple forms of the target nucleic acid are detected.

4. The system according to claim 1, wherein the hapten is biotin, fluorophore or oligopeptide.

5. The system according to claim 4, wherein said specific binding partner is streptavidin, hapten specific antibody, or oligonucleotide complementary to the nucleic acid complex.

6. The system according to claim 5, wherein the reporter dye is europium, gold or fluorophore.

7. The system according to claim 3, wherein the multiple type of hapten comprises multiple types of fluorophores or oligopeptides.

8. The system according to claim 1, wherein immobilized in the test area is a specific binding partner that is different from the specific binding partner in the dye area.

9. The system according to claim 8, wherein said specific partner immobilized in the test area is an antibody to a hapten and/or a complementary oligonucleotide or PNA to the target nucleic acid.

10. The system according to claim 9, wherein said specific binding partner immobilized on the test area is DNA.

11. The system according to claim 10, wherein said lateral flow device comprises connectors to a device for assaying or reading electroconductivity.

12. The system according to claim 1, wherein the source of the target nucleic acid is a pathogen.

13. The system according to claim 12, wherein the pathogen is a virus belonging to family Flaviviridae.

14. The system according to claim 13, wherein the virus is dengue virus.

15. The system according to claim 14, wherein serotypes of dengue virus particles are detected by labeling primers with multiple different haptens to distinguish them.

16. The system according to claim 1, wherein the primer is labeled at its one end with a fluorophore and its other end with a quencher and the dNTP is labeled with biotin.

17. A method of assaying for the presence of one or more target nucleic acids in a sample, comprising using an immunochromatographic system for detecting one or more target nucleic acids said system comprising:
a nucleic acid amplification mix comprising
a primer labeled with a hapten or different haptens at its 5' and 3' ends, and
dNTP labeled with a signal amplifying hapten to form one or more nucleic acid complexes and
a lateral flow test device comprising
a reservoir area comprising reagents that are suitable for binding of a specific binding partner with the one or more nucleic acid complexes;
a dye area, adjacent to the reservoir area, comprising a specific binding partner to a hapten on the primer or the hapten on the one or more nucleic acid complexes wherein the specific binding partner is linked or conjugated to a reporter dye or another hapten; and
a test area, adjacent to the dye area, comprising a different specific
binding partner specific to a different aspect of the one or more nucleic acid complexes, comprising the steps of:
(i) contacting the sample to the nucleic acid amplification mix comprising a primer labeled with a hapten or different haptens at its 5' and 3' ends, and dNTP labeled with a signal amplifying hapten to form one or more nucleic acid complexes,
(ii) contacting the amplified nucleic acid obtained thereby to the reservoir area of the lateral flow device; and
(iii) assaying for the binding of the nucleic acid complex to the specific binding partner on the test area, wherein the dye area comprises target nucleic acid specific oligonucleotide labeled with a reporter dye and the labeled oligonucleotide binds to the one or more target nucleic acid forming a nucleic acid complex, and wherein the nucleic acid complex flows to the test area, wherein the test area comprises a specific binding partner specific to an aspect of the nucleic acid complex, wherein binding of the nucleic acid complex to its specific binding partner results in a positive signal for the presence of the target nucleic acid.

18. The method according to claim 17, wherein the signal is read by reporter dye or electroconductivity.

19. The system according to claim 1, wherein the container comprises MgCl₂ in a concentration not higher than 1.5mM.

20. The method according to claim 17, wherein the container comprises MgCl₂ in a concentration not higher than 1,5 mM.

## Patentansprüche

1. Immunochromatographisches System zum Nachweis von einer oder mehreren Ziel-Nukleinsäuren, umfassend:
einen Behälter mit einem Nukleinsäure-Amplifikationsgemisch, enthaltend
einen Primer, der an seinen 5'- und 3'-Enden mit einem Hapten oder unterschiedlichen Haptenen markiert ist, sowie dNTP, markiert mit einem signalamplifizierenden Hapten, um einen oder mehrere Nukleinsäure-Komplexe zu bilden; und
eine Lateralfluss-Testvorrichtung, umfassend
eine Reservoirzone mit Reagentien, die geeignet sind, einen spezifischen Bindungspartner mit einem oder mehreren Nukleinsäure-Komplexen zu binden;
einen Farbstoffbereich benachbart zur Reservoirzone, enthaltend einen spezifischen Bindungspartner zu einem Hapten am Primer oder dem Hapten an einen oder mehreren Nukleinsäure-Komplexen, wobei der spezifische Bindungspartner an einen Reporter-Farbstoff oder ein anderes Hapten gebunden oder konjugiert ist; und
einen zum Farbstoffbereich benachbarten Testbereich, enthaltend einen anderen spezifischen Bindungspartner, der spezifisch zu einem anderen Aspekt des einen oder der mehreren Nukleinsäure-Komplexe ist.

2. System nach Anspruch 1, wobei das Nukleinsäure-Amplifikationsgemisch getrocknet oder gefriergetrocknet ist und geeignet zur isothermen Amplifikation, PCR und/oder Echtzeit-PCR ist.

3. System nach Anspruch 1, wobei das Nukleinsäure-Amplifikationsgemisch Mehrfach-Arten von Hapten umfasst, an denen mehrere Formen der Ziel-Nukleinsäure nachgewiesen werden.

4. System nach Anspruch 1, wobei das Hapten Biotin, Fluorophor oder Oligopeptid ist.

5. System nach Anspruch 4, wobei der spezifische Bindungspartner Streptavidin, Haptenspezifischer Antikörper oder Oligonukleotid, komplementär zum Nukleinsäure-Komplex ist.

6. System nach Anspruch 5, wobei der Reporterfarbstoff Europium, Gold oder Fluorophor ist.

7. System nach Anspruch 3, wobei die Mehrfach-Arten von Hapten mehrere Typen von Fluorophoren oder Oligopeptiden umfassen.

8. System nach Anspruch 1, wobei im Testbereich ein spezifischer Bindungspartner immobilisiert ist, der sich vom spezifischen Bindungspartner im Farbstoffbereich unterscheidet.

9. System nach Anspruch 8, wobei der im Testbereich immobilisierte, spezifische Partner ein Antikörper zu einem Hapten und/oder ein komplementäres Oligonukleotid oder PNA zur Ziel-Nukleinsäure ist.

10. System nach Anspruch 9, wobei der im Testbereich immobilisierte, spezifische Bindungspartner DNA ist.

11. System nach Anspruch 10, wobei die Lateralflussvorrichtung Anschlüsse zu einem Testgerät oder Messgerät für die elektrische Leitfähigkeit aufweist.

12. System nach Anspruch 1, wobei die Quelle der Ziel-Nukleinsäure ein Pathogen ist.

13. System nach Anspruch 12, wobei das Pathogen ein Virus der Familie Flaviviridae ist.

14. System nach Anspruch 13, wobei das Virus Dengue-Virus ist.

15. System nach Anspruch 14, wobei Serotypen der Dengue-Virus-Partikel von Primern detektiert werden, die zur Unterscheidung mit mehreren unterschiedlichen Haptenen markiert sind.

16. System gemäß Anspruch 1, wobei der Primer an einem Ende mit einem Fluorophor und am anderen Ende mit einem Quencher markiert ist, und die dNTP mit Biotin markiert ist.

17. Verfahren zum Testen auf Vorhandensein einer oder mehrerer Ziel-Nukleinsäuren in einer Probe, umfassend ein immunochromatographisches System zum Nachweis von einer oder mehreren Ziel-Nukleinsäuren, umfassend:
ein Nukleinsäure-Amplifikationsgemisch, enthaltend
einen Primer, der an seinen 5'- und 3'-Enden mit einem Hapten oder unterschiedlichen Haptenen markiert ist, sowie dNTP, markiert mit einem signalamplifizierenden Hapten, um einen oder mehrere Nukleinsäure-Komplexe zu bilden; und
eine Lateralfluss-Testvorrichtung, umfassend
eine Reservoirzone mit Reagentien, die geeignet sind, einen spezifischen Bindungspartner mit einem oder mehreren Nukleinsäure-Komplexen zu binden;
einen Farbstoffbereich benachbart zur Reservoirzone, enthaltend einen spezifischen Bindungspartner zu einem Hapten am Primer oder dem Hapten an einen oder mehreren Nukleinsäure-Komplexen, wobei der spezifische Bindungspartner an einen Reporter-Farbstoff oder ein anderes Hapten gebunden oder konjugiert ist; und
einen zum Farbstoffbereich benachbarten Testbereich, enthaltend einen anderen spezifischen Bindungspartner, der spezifisch zu einem anderen Aspekt des einen oder der mehreren Nukleinsäure-Komplexe ist,
umfassend die Schritte:
(i) Kontaktieren der Probe mit dem Nukleinsäure-Amplifikationsgemisch, enthaltend einen Primer, der an seinen 5'- und 3'-Enden mit einem Hapten oder unterschiedlichen Haptenen markiert ist, sowie dNTP, markiert mit einem signalamplifizierenden Hapten, um einen oder mehrere Nukleinsäure-Komplexe zu bilden;
(ii) Kontaktieren der so erhaltenen, amplifizierten Nukleinsäure mit der Reservoirzone der Lateralfluss-Testvorrichtung, und
(iii) Testen der Bindung des Nukleinsäure-Komplexes zum spezifischen Bindungspartner im Testbereich, wobei der Farbstoffbereich Ziel-Nukleinsäure spezifische Oligonukleotide enthält, die mit einem Reporter- Farbstoff markiert sind, und das markierte Oligonukleotid sich an die eine oder mehrere Ziel-Nukleinsäuren bindet, um einen Nukleinsäure-Komplex zu bilden, und der Nukleinsäure-Komplex zum Testbereich fließt, wobei der Testbereich einen spezifischen Bindungspartner enthält, der spezifisch zu einem Aspekt des Nukleinsäure-Komplexes ist, wobei die Bindung des Nukleinsäure-Komplexes an seinen spezifischen Bindungspartner ein positives Signal für das Vorhandensein der Ziel-Nukleinsäure ergibt.

18. Verfahren nach Anspruch 17, wobei das Signal durch Reporter-Farbstoff oder elektrische Leitfähigkeit gelesen wird.

19. System nach Anspruch 1, wobei der Behälter MgCl₂ in einer Konzentration von nicht höher als 1,5 mM enthält.

20. Verfahren nach Anspruch 17, wobei der Behälter MgCl₂ in einer Konzentration von nicht höher als 1,5 mM enthält.

## Revendications

1. Système immunochromatographique pour la détection d'un ou de plusieurs acides nucléiques cibles, ledit système comprenant :
un récipient comprenant un mélange d'amplification d'acide nucléique comprenant
une amorce marquée avec un haptène ou des haptènes différents à ses extrémités 5' et 3', et
des dNTP marqués avec un haptène amplifiant le signal pour former un ou plusieurs complexes d'acide nucléique ; et
un dispositif de test à flux latéral comprenant
une zone de réservoir comprenant des réactifs qui sont appropriés à la liaison d'un partenaire de liaison spécifique avec les un ou plusieurs complexes d'acide nucléique ;
une zone de colorant, adjacente à la zone du réservoir, comprenant un partenaire de liaison spécifique à un haptène sur l'amorce ou à l'haptène sur les un ou plusieurs complexes d'acide nucléique, où le partenaire de liaison spécifique est lié ou conjugué à un colorant rapporteur ou à un autre haptène ; et
une zone de test, adjacente à la zone du colorant, comprenant un partenaire de liaison spécifique différent spécifique d'un aspect différent des un ou plusieurs complexes d'acide nucléique.

2. Système selon la revendication 1, dans lequel le mélange d'amplification d'acide nucléique est séché ou lyophilisé et est approprié pour une amplification isotherme, une PCR et/ou une PCR en temps réel.

3. Système selon la revendication 1, dans lequel le mélange d'amplification d'acide nucléique comprend des types multiples d'haptène où des formes multiples de l'acide nucléique cible sont détectées.

4. Système selon la revendication 1, dans lequel l'haptène est la biotine, un fluorophore ou un oligopeptide.

5. Système selon la revendication 4, dans lequel ledit partenaire de liaison spécifique est la streptavidine, un anticorps spécifique de l'haptène, ou un oligonucléotide complémentaire du complexe d'acide nucléique.

6. Système selon la revendication 5, dans lequel le colorant rapporteur est l'europium, l'or ou un fluorophore.

7. Système selon la revendication 3, dans lequel le type multiple d'haptène comprend des types multiples de fluorophores ou d'oligopeptides.

8. Système selon la revendication 1, dans lequel est immobilisé dans la zone de test un partenaire de liaison spécifique qui est différent du partenaire de liaison spécifique dans la zone de colorant.

9. Système selon la revendication 8, dans lequel ledit partenaire spécifique immobilisé dans la zone de test est un anticorps dirigé contre un haptène et/ou un oligonucléotide ou un PNA complémentaire de l'acide nucléique cible.

10. Système selon la revendication 9, dans lequel ledit partenaire de liaison spécifique immobilisé sur la zone de test est de l'ADN.

11. Système selon la revendication 10, dans lequel ledit dispositif à flux latéral comprend des connecteurs à un dispositif d'analyse ou de lecture de l'électroconductivité.

12. Système selon la revendication 1, dans lequel la source de l'acide nucléique cible est un agent pathogène.

13. Système selon la revendication 12, dans lequel l'agent pathogène est un virus appartenant à la famille des Flaviviridés.

14. Système selon la revendication 13, dans lequel le virus est le virus de la dengue.

15. Système selon la revendication 14, dans lequel les sérotypes des particules du virus de la dengue sont détectés par marquage des amorces avec de multiples haptènes différents pour les distinguer.

16. Système selon la revendication 1, dans lequel l'amorce est marquée à l'une de ses extrémités avec un fluorophore et à l'autre de ses extrémités avec un agent d'extinction et le dNTP est marqué avec de la biotine.

17. Procédé d'analyse de la présence d'un ou de plusieurs acides nucléiques cibles dans un échantillon, comprenant l'utilisation d'un système immunochromatographique pour la détection des un ou plusieurs acides nucléiques cibles, ledit système comprenant :
un mélange d'amplification d'acide nucléique comprenant
une amorce marquée avec un haptène ou des haptènes différents à ses extrémités 5' et 3', et
des dNTP marqués avec un haptène amplifiant le signal pour former un ou plusieurs complexes d'acide nucléique ; et
un dispositif de test à flux latéral comprenant
une zone de réservoir comprenant des réactifs qui sont appropriés à la liaison d'un partenaire de liaison spécifique avec les un ou plusieurs complexes d'acide nucléique ;
une zone de colorant, adjacente à la zone du réservoir, comprenant un partenaire de liaison spécifique à un haptène sur l'amorce ou à l'haptène sur les un ou plusieurs complexes d'acide nucléique, où le partenaire de liaison spécifique est lié ou conjugué à un colorant rapporteur ou à un autre haptène ; et
une zone de test, adjacente à la zone du colorant, comprenant un partenaire de liaison spécifique différent spécifique d'un aspect différent des un ou plusieurs complexes d'acide nucléique,
comprenant les étapes suivantes :
(i) la mise en contact de l'échantillon avec le mélange d'amplification d'acide nucléique comprenant une amorce marquée avec un haptène ou des haptènes différents à ses extrémités 5' et 3', et des dNTP marqués avec un haptène amplifiant le signal pour former un ou plusieurs complexes d'acide nucléique ;
(ii) la mise en contact de l'acide nucléique amplifié obtenu de cette façon avec la zone de réservoir du dispositif à flux latéral ; et
(iii) l'analyse de la liaison du complexe d'acide nucléique au partenaire de liaison spécifique sur la zone de test, où la zone de colorant comprend un oligonucléotide spécifique de l'acide nucléique cible marqué avec un colorant rapporteur et l'oligonucléotide marqué se lie aux un ou plusieurs acides nucléiques cibles formant un complexe d'acide nucléique, et où le complexe d'acide nucléique circule sur la zone de test, où la zone de test comprend un partenaire de liaison spécifique, spécifique d'un aspect du complexe d'acide nucléique, où la liaison du complexe d'acide nucléique à son partenaire de liaison spécifique se traduit par un signal positif pour la présence de l'acide nucléique cible.

18. Procédé selon la revendication 17, dans lequel le signal est lu par le colorant rapporteur ou l'électroconductivité.

19. Système selon la revendication 1, dans lequel le récipient comprend du MgCl₂ dans une concentration qui n'est pas supérieure à 1,5 mM.

20. Procédé selon la revendication 17, dans lequel le récipient comprend du MgCl₂ dans une concentration qui n'est pas supérieure à 1,5 mM.
